# EUROPEAN PATENT SPECIFICATION

(11) **EP 0 560 482 B1**
(45) Date of publication and mention of the grant of the patent: **06.08.1997**
(21) Application number: 93300816.1
(22) Date of filing: 04.02.1993
(51) Int. Cl.: C07D 261/08, C07D 261/18, A01N 43/80

(54) **4-Benzoyl isoxazole derivatives and their use as herbicides**
4-Benzoylisoxazol Derivate und ihre Verwendung als Herbizide
Dérivés de 4-benzoyl isoxazole et leur utilisation comme herbicides

(30) Priority: 12.03.1992 US 850128
(43) Date of publication of application: 15.09.1993
(73) Proprietor: RHONE-POULENC AGRICULTURE LTD., Ongar, Essex CM5 0HW (GB)
(72) Inventor: Cain, Paul Alfred, Ongar, Essex CM5 0HW (GB); Cramp, Susan Mary, Ongar, Essex CM5 0HW (GB)
(74) Representative: Bentham, Stephen

(56) References cited:
- EP-A- 487 357
- EP-A- 527 036
- EP-A- 527 037
- EP-A- 0 418 175

## Description

This invention relates to novel 4-benzoylisoxazole derivatives, compositions containing them and their use as herbicides.

The following four applications describe 4-benzoyl isoxazoles having herbicidal properties in which the isoxazole ring and the phenyl ring of the benzoyl group are substituted:
in EP-A-418175 the isoxazole ring is substituted in the 5-position and the phenyl ring carries 1 to 5 substituents;
in EP-A-487357, published after the priority date of the present application, the isoxazole ring is substituted in the 3-position and optionally in the 5-position and the phenyl ring carries 1 to 5 substituents;
in EP-A-527036, published after the filing date of the present application, the isoxazole ring is substituted in the 5-position by cyclopropyl and the phenyl ring is 2,4-disubstituted with a group -SO₂Me in the 2-position;
in EP-A-527037, published after the filing date of the present application, the isoxazole is substituted in the 5-position and the phenyl ring is 2,4-disubstituted with an optional substituent on the 3-position. The 4-substituent is -S(O)ₙR (n=0, 1 or 2 and R = methyl or ethyl). The present invention provides 4-benzoylisoxazole derivatives of general formula (I): 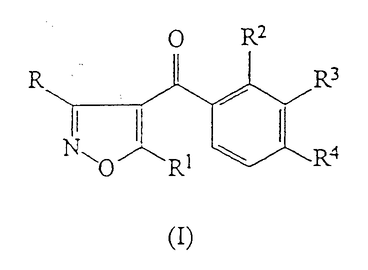 wherein
   R represents a hydrogen atom or a group -CO₂R⁵;
   R¹ represents cyclopropyl;
   R² represents -S(O)ₙR⁵¹;
   R³ represents:
      a chlorine, bromine or fluorine atom;
      a straight- or branched- chain alkyl or alkoxy group containing up to four carbon atoms optionally substituted by one or more halogen atoms;
      a straight- or branched chain alkenyl group containing up to six carbon atoms; or
      a group -CO₂R⁵²;
   R⁴ represents:
      a chlorine, bromine or fluorine atom;
      a straight- or branched- chain alkyl group containing up to four carbon atoms optionally substituted by one or more halogen atoms;
      an alkoxy group containing up to four carbon atoms substituted by one or more halogen atoms;
      -S(O)ₚR⁵³ or cyano;
   R⁵ represents a straight- or branched- chain alkyl group containing up to six carbon atoms optionally substituted by one or more halogen atoms;
   R⁵¹ represents methyl or ethyl;
   R⁵² represents methyl or ethyl;
   R⁵³ represents a straight- or branched- chain alkyl group containing up to four carbon atoms;
   n represents zero, one or two; and
   p represents zero, one or two.

In certain cases the substituents R¹, R² R³, R⁴, R⁵, R⁵¹ and R⁵³ contribute to optical and/or stereoisomerism. All such forms are embraced by the present invention.

The compounds of the invention show unexpected and remarkably high herbicidal activity in comparison with known compounds against important weed species including foxtail (Setaria viridis and Setaria faberii), barnyard grass (Echinochloa crus-galli), crabgrass (Digitaria sanguinalis) and shattercane (Sorghum bicolor).

Where R represents -CO₂R⁵, R⁵ is preferably methyl or ethyl.

Where R³ represents an alkenyl group, the alkenyl group preferably contains from two to four carbon atoms, more preferably two or three carbon atoms. Where R³ represents -CO₂R⁵², R⁵² is preferably methyl. Where R³ represents a halogen-substituted alkyl group, preferably R³ is not trifluoromethyl; preferred halogen-substituted alkyl groups include for example difluoromethyl, 2,2,2-trifluoroethyl, fluoromethyl and dichlorofluoromethyl. Preferred compounds include those wherein R³ represents an optionally halogen-substituted alkoxy group containing one or two carbon atoms, more preferably ethoxy or most preferably methoxy.

Where R⁴ represents -S(O)ₚR⁵³, preferably p is zero and/or R⁵³ is ethyl or most preferably methyl.

Compounds in which R⁵¹ represents methyl are also preferred.

A preferred class of compounds of general formula (I) are those wherein:
R³ represents a fluorine, chlorine or bromine atom; a methyl or ethyl group; an alkoxy group containing one or two carbon atoms optionally substituted by one or more halogen atoms; an alkenyl group containing from two to four carbon atoms; or -CO₂R⁵²;
R⁴ represents a fluorine, chlorine or bromine atom; an alkyl group containing one or two carbon atoms substituted by one or more halogen atoms; an alkoxy group containing one or two carbon atoms substituted by one or more halogen atoms; or -S(O)ₚR⁵³, wherein p represents zero and R⁵³ is a methyl or ethyl group; and
R⁵¹ represents a methyl or ethyl group.

A further preferred class of compounds of general formula (I) are those wherein:
R³ represents a fluorine, chlorine or bromine atom; a methyl, methoxy or ethoxy group; an alkenyl group containing two or three carbon atoms; or -CO₂R⁵² wherein R⁵² is methyl;
R⁴ represents a fluorine, chlorine or bromine atom or a group selected from trifluoromethyl, trifluoromethoxy and -S(O)ₚMe wherein p is zero;
R⁵ represents a methyl or ethyl group; and
R⁵¹ represents a methyl or ethyl group.

A further preferred class of compounds of general formula (I) are those wherein:
R³ is fluorine, chlorine, bromine, methyl or methoxy;
R⁴ is fluorine, chlorine, bromine or trifluoromethyl;
R⁵ is methyl or ethyl.

A further preferred class of compounds of general formula (I) are those wherein:
R³ is fluorine, chlorine, bromine or methoxy;
R⁴ is fluorine, chlorine, bromine or trifluoromethyl;
R⁵ is methyl or ethyl; and
R⁵¹ is methyl.

A further preferred class of compounds of general formula (I) are those wherein:
R³ represents a chlorine, bromine or fluorine atom;
R⁴ represents fluorine, chlorine, bromine or trifluoromethyl;
R⁵ represents methyl or ethyl; and
R⁵¹ represents methyl.

Compounds of particular interest because of their herbicidal activity include the following:
1. 5-cyclopropyl-4-[3,4-difluoro-2-(methylsulphonyl)benzoyl]isoxazole;
2. 5-cyclopropyl-4-[3,4-dichloro-2-(methylsulphenyl)benzoyl]isoxazole;
3. 5-cyclopropyl-4-[3,4-dichloro-2-(methylsulphinyl)benzoyl]isoxazole;
4. 5-cyclopropyl-4-[3,4-dichloro-2-(methylsulphonyl)benzoyl]isoxazole;
5. 5-cyclopropyl-4-[4-bromo-3-methoxy-2-(methylsulphenyl)benzoyl]isoxazole;
6. 5-cyclopropyl-4-[4-bromo-3-methoxy-2-(methylsulphonyl)benzoyl]isoxazole;
7. 5-cyclopropyl-4-[4-bromo-3-methoxy-2-(methylsulphinyl)benzoyl]isoxazole;
8. ethyl 5-cyclopropyl-4-[3,4-dichloro-2-(methylsulphenyl)benzoyl]isoxazole-3-carboxylate;
9. ethyl 5-cyclopropyl-4-[3,4-dichloro-2-(methylsulphinyl)benzoyl]isoxazole-3-carboxylate;
10. 4-[4-chloro-3-methoxy-2-(methylsulphenyl)benzoyl]-5-cyclopropylisoxazole;
11. ethyl 5-cyclopropyl-4-[3,4-dichloro-2-(methylsulphonyl)benzoyl]isoxazole-3-carboxylate;
12. 4-[4-chloro-3-methoxy-2-(methylsulphinyl)benzoyl]-5-cyclopropylisoxazole;
13. 4-[4-chloro-3-methoxy--(methylsulphonyl)benzoyl]-5-cyclopropylisoxazole;
14. 4-[4-chloro-3-methyl-2-(methylsulphenyl)benzoyl]-5-cyclopropylisoxazole;
15. 4-[4-chloro-3-fluoro-2-(methylsulphenyl)benzoyl]-5-cyclopropylisoxazole;
16. 4-[4-chloro-3-fluoro-2-(methylsulphinyl)benzoyl]-5-cyclopropylisoxazole;
17. 4-[4-chloro-3-fluoro-2-(methylsulphonyl)benzoyl]-5-cyclopropylisoxazole;
18. 4-[4-chloro-3-methyl-2-(methylsulphinyl)benzoyl]-5-cyclopropylisoxazole;
19. 4-[4-chloro-3-methyl-2-(methylsulphonyl)benzoyl]-5-cyclopropylisoxazole;
20. 5-cyclopropyl-4-[3-methoxycarbonyl-2-(methylsulphenyl)-4-trifluoromethylbenzoyl]isoxazole;
21. 4-[4-chloro-3-methoxycarbonyl-2-(methylsulphenyl)benzoyl]-5-cyclopropylisoxazole;
22. 4-[4-bromo-3-chloro-2-(methylsulphenyl)benzoyl]-5-cyclopropylisoxazole;
23. 4-[4-bromo-3-chloro-2-(methylsulphinyl)benzoyl]-5-cyclopropylisoxazole;
24. 4-[4-bromo-3-chloro-2-(methylsulphonyl)benzoyl]-5-cyclopropylisoxazole;
25. 4-[4-chloro-3-methoxycarbonyl-2-(methylsulphinyl)benzoyl]-5-cyclopropylisoxazole;
26. 4-[4-chloro-3-methoxycarbonyl-2-(methylsulphonyl)benzoyl]-5-cyclopropylisoxazole;
27. 4-[3-chloro-2-(methylsulphenyl)-4-trifluoromethylbenzoyl]-5-cyclopropylisoxazole;
28. 4-[3-chloro-2-(methylsulphonyl)-4-trifluoromethylbenzoyl]-5-cyclopropylisoxazole;
29. 4-[4-bromo-3-fluoro-2-(methylsulphenyl)benzoyl]-5-cyclopropylisoxazole;
30. 4-[4-bromo-3-fluoro-2-(methylsulphinyl)benzoyl]-5-cyclopropylisoxazole;
31. 4-[4-bromo-3-fluoro-2-(methylsulphonyl)benzoyl]-5-cyclopropylisoxazole;
32. 4-[4-chloro-3-isopropenyl-2-(methylsulphenyl)benzoyl]-5-cyclopropylisoxazole;
33. 5-cyclopropyl-4-[3-methyl-2,4-bis(methylsulphenyl)benzoyl]isoxazole;
34. 4-[4-chloro-3-isopropenyl-2-(methylsulphinyl)-benzoyl]-5-cyclopropylisoxazole; and
35. 4-[4-chloro-3-isopropenyl-2-(methylsulphonyl)benzoyl]-5-cyclopropylisoxazole.

The numbers 1 to 35 are assigned to these compounds for reference and identification hereinafter.

Of the above, compounds 3, 4, 6, 8, 13, 16, 17, 22, 23 and 24 are particularly preferred.

Compounds of general formula (I) may be prepared by the application or adaptation of known methods (i.e. methods heretofore used or described in the literature), for example as hereinafter described.

In the following description where symbols appearing in formulae are not specifically defined, it is to be understood that they are "as hereinbefore defined" in accordance with the first definition of each symbol in the specification.

It is to be understood that in the descriptions of the following processes the sequences may be performed in different orders, and that suitable protecting groups may be required to achieve the compounds sought.

According to a feature of the present invention compounds of general formula (I) in which R represents hydrogen may be prepared by the reaction of a compound of general formula (II): 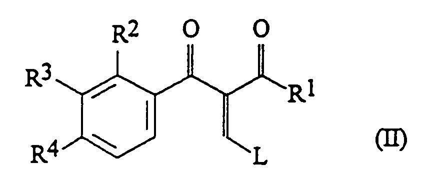 wherein R¹, R², R³ and R⁴ are as hereinbefore defined and L is a leaving group, with a salt of hydroxylamine. Hydroxylamine hydrochloride is generally preferred. Generally L is alkoxy, for example ethoxy, or N,N-dialkylamino, for example dimethylamino. The reaction is generally carried out in a solvent such as ethanol or acetonitrile, optionally in the presence of a base or acid acceptor such as triethylamine or sodium acetate.

According to a further feature of the present invention compounds of general formula (I) in which R² represents a group -SR⁵¹, R represents hydrogen and R⁴ represents a group R⁴¹ which is as hereinbefore defined for R⁴ provided that p is zero, may be prepared by the reaction of a compound of general formula (III): 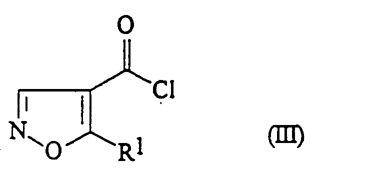 wherein R¹ is as hereinbefore defined, with a compound of general formula (IV): 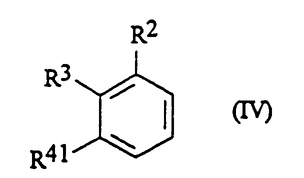 in which R³ and R⁴¹ are as hereinbefore defined and R² represents -SR⁵¹. The reaction is generally carried out in the presence of a Lewis acid catalyst such as aluminium chloride at a temperature between room temperature and 100°C.

According to a further feature of the present invention compounds of general formula (I) in which R represents hydrogen may be prepared by the reaction of a compound of general formula (V): 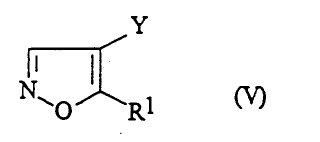 wherein R¹ is as hereinbefore defined and Y represents a carboxy group or a reactive derivative thereof (such as a carboxylic acid chloride or carboxylic ester), or a guano group, with an appropriate organometallic reagent such as a Grignard reagent or an organolithium reagent. The reaction is generally carried out in an inert solvent such as ether or tetrahydrofuran at a temperature from 0°C to the reflux temperature of the mixture.

According to a further feature of the invention compounds of formula (I) wherein R represents a group -CO₂R⁵, n is 0 or 2 and R⁴ represents a group R⁴² which is as hereinbefore defined for R⁴ provided that p is 0 or 2, may be prepared by the reaction of a compound of general formula (VI): 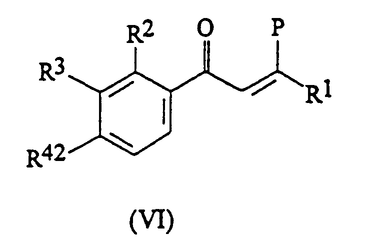 wherein R¹, R², R³ and R⁴² are as hereinbefore defined, n is zero or two and P is a leaving group such as N,N-dialkylamino, with a compound of general formula R⁵O₂CC(X)=NOH wherein R⁵ is as hereinbefore defined and X is a halogen atom. Generally X is chlorine or bromine. The reaction is generally performed in an inert solvent such as toluene or dichloromethane either in the presence of a base such as triethylamine or a catalyst such as a 4 Angstrom molecular sieve or fluoride ion.

According to a further feature of the present invention compounds of general formula I in which R represents a group -CO₂R⁵, n is 0 or 2 and R⁴ represents a group R⁴² as hereinbefore defined, may be prepared by the reaction of a compound of general formula (VII): 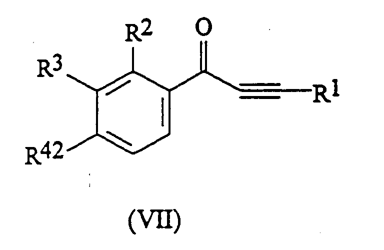 wherein R¹, R², R³ and R⁴² are as hereinbefore defined and n is 0 or 2, with a compound of general formula R⁵O₂CC(X)=NOH, wherein R⁵ and X are as hereinbefore defined. The reaction is generally performed in an inert solvent such as toluene or dichloromethane optionally in the presence of a base such as triethylamine or a catalyst such as a 4 Angstrom molecular sieve or fluoride ion. The reaction can be carried out at a temperature between room temperature and the reflux temperature of the mixture.

According to a further feature of the present invention compounds of general formula (I) wherein R represents -CO₂R⁵, n is 0 or 2 and R⁴ represents a group R⁴² as hereinbefore defined, may be prepared by the reaction of a salt of a compound of general formula (VIII): 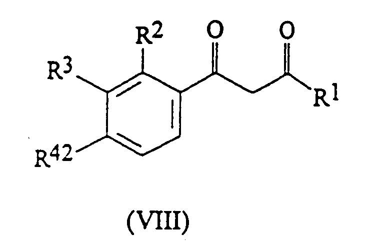 wherein R¹, R², R³ and R⁴² are as hereinbefore defined and n is 0 or 2, with a compound of general formula R⁵O₂CC(X)=NOH wherein R⁵ and X are as hereinbefore defined. Preferred salts include sodium or magnesium salts. The reaction may be performed in an inert solvent such as dichloromethane or acetonitrile at a temperature between room temperature and the reflux temperature of the mixture.

Intermediates in the preparation of compounds of general formula (I) may be prepared by the application or adaptation of known methods.

Compounds of general formula (II) may be prepared by the reaction of compounds of general formula (VIII) with either a trialkyl orthoformate such as triethyl orthoformate or a dimethylformamide dialkylacetal such as dimethylformamide dimethyl acetal.

The reaction with triethyl orthoformate is generally carried out in the presence of acetic anhydride at the reflux temperature of the mixture and the reaction with dimethylformamidedialkyl acetal is carried out optionally in the presence of an inert solvent at a temperature from room temperature to the reflux temperature of the mixture.

Compounds of general formula (VI) may be prepared by the reaction of a compound of general formula (IX) wherein R¹ and P are as hereinbefore defined with a benzoyl chloride of general formula (X) wherein R², R³ and R⁴² are as hereinbefore defined:

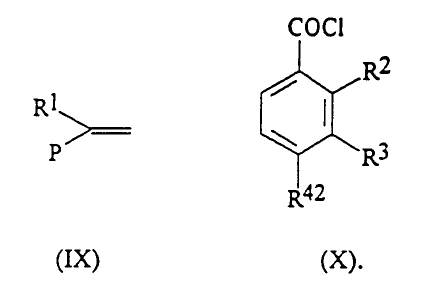

The reaction is generally carried out in the presence of an organic base such as triethylamine in an inert solvent such as toluene or dichloromethane at a temperature between -20°C and room temperature.

Compounds of general formula (VII) may be prepared by the metallation of the appropriate acetylene of general formula (XI):

R¹―C≡CH (XI)

wherein R¹ is as hereinbefore defined, followed by reaction of the metal salt thus obtained with a benzoyl chloride of general formula (X). The metallation is generally performed using n-butyl lithium in an inert solvent such as ether or tetrahydrofuran at a temperature from -78°C to 0°C. The subsequent reaction with the benzoyl chloride is carried out in the same solvent at a temperature between -78°C and room temperature.

Those skilled in the art will appreciate that some compounds of general formula (I) may be prepared by the interconversion of other compounds of general formula (I) and such interconversions constitute yet more features of the present invention. Examples of such interconversions are hereafter described.

According to a further feature of the present invention compounds in which n is one or two and/or p is one or two may be prepared by the oxidation of the sulphur atom of compounds in which n is zero or one and/or p is zero or one . The oxidation of the sulphur atom is generally carried out using for example 3-chloroperoxybenzoic acid in an inert solvent such as dichloromethane at a temperature from -40°C to room temperature, or hydrogen peroxide in acetic acid in the presence of acetic anhydride or concentrated sulphuric acid.

Benzoic acids required as intermediates in the preparation of compounds of general formula I may be prepared according to a number of processes for example as hereinafter described.

Benzoic acids or esters of general formula XII may be prepared by diazotization of compounds of general formula XIII followed by treatment with a dialkyl disulphide, R⁵¹S-SR⁵¹: 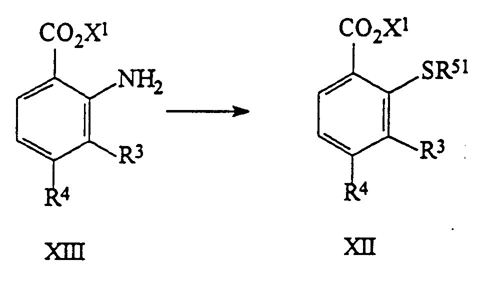 wherein R³, R⁴ and R⁵¹ are as hereinbefore defined and X¹ represents hydrogen, methyl or ethyl. Diazotization may be performed using an alkyl nitrite such as t-butyl nitrite in the presence of a dialkyl disulphide in an inert solvent such as chloroform at a temperature from room temperature to the reflux temperature of the mixture. Alternatively diazotization may be carried out using sodium nitrite followed by treatment with a dialkyl disulphide in the presence of a catalyst such as copper.

Alternatively benzoic acids or esters of general formula XII may be prepared from compounds of general formula XIV: 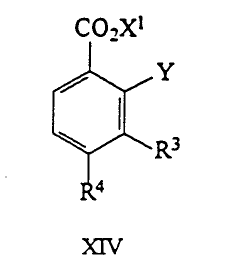 wherein R³, R⁴ and X¹ are as hereinbefore described and Y is a halogen atom (e.g. chlorine, fluorine or bromine) or a nitro group, with an alkyl mercaptan of formula R⁵¹-SH wherein R⁵¹ is as hereinbefore defined, in the presence of a base. Typical bases used in the above reaction include lithium hydroxide and potassium carbonate and the reaction may be carried out in a solvent such as dimethyl formamide or acetone at a temperature from room temperature to the reflux temperature of the mixture.

Alternatively benzoic acids of general formula XII in which R³ represents a halogen atom may be prepared by lithiation of compounds of general formula XV to give the lithiated intermediate XVa: 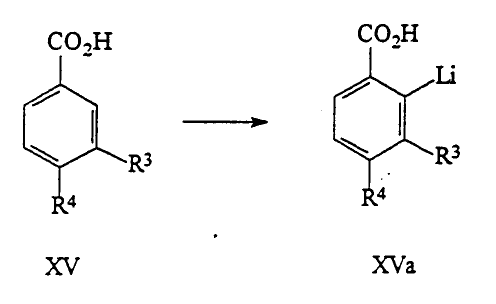 wherein R⁴ is as hereinbefore defined and R³ represents a halogen atom, which is treated with a dialkyl disulphide, R⁵¹S-SR⁵¹, wherein R⁵¹ is as hereinbefore defined. The lithiation is typically carried out using alkyl lithium compounds such as n-butyl lithium or lithium diisopropylamide in an inert solvent such as tetrahydrofuran at a temperature from -70°C to -40°C. The reaction is preferably performed under an inert atmosphere. This reaction, giving the lithiated intermediate (XVa) is novel and as such constitutes a further feature of the present invention.

The benzoic acids of general formula (XII) may also be prepared from benzoic acids of general formula (XV) by first protecting the benzoic acid function as a 4,4-dimethyloxazoline to give a compound of general formula (XVI): 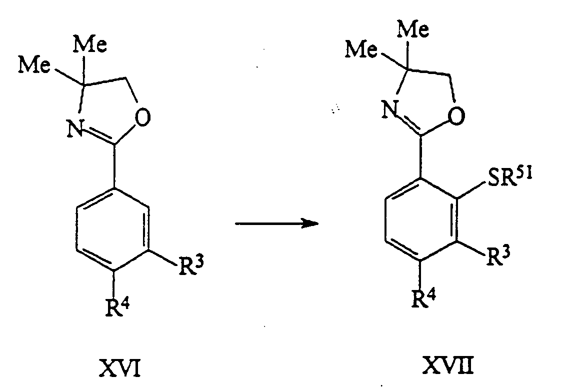 wherein R³ and R⁴ are as hereinbefore defined, which is then lithiated using for example n-butyllithium or lithium diisopropyl amide followed by treatment with a dialkyl disulphide of formula R⁵¹S-SR⁵¹, wherein R⁵¹ is as hereinbefore defined. Compounds of formula XVI are described in the literature, for example by A Metikian et al, Eur. J. Med. Chem. 25 (1990) 267-270. The oxazoline of general formula XVII is then converted to the benzoic acid as described for example by A.I. Meyers J.Org.Chem.40 (1975) 3158-3159.

Intermediates of general formula (III), (IV), (V), (VIII), (IX), (X), (XI), (XIII), (XIV) and (XV) are known or may be prepared by the application or adaptation of known methods.

The following examples illustrate the preparation of compounds of general formula (I) and the following reference examples illustrate the preparation of intermediates of the invention. In the present specification b.p. means boiling point; m.p. means melting point. Where the letters NMR appear the characteristics of the proton nuclear magnetic resonance spectrum follow.

### Example 1

Sodium acetate (0.31g) was added with stirring to a mixture of 3-cyclopropyl-1-[3,4-difluoro-2-(methylsulphonyl)phenyl]-2-ethoxymethylene-propane-1,3-dione (1.1g) and hydroxylamine hydrochloride (0.26g) in ethanol. The mixture was stirred for 2.5 hours. The mixture was evaporated to dryness and the residue was suspended in ethyl acetate, washed with water, dried (anhydrous MgSO₄) and filtered. The filtrate was evaporated to dryness. The residue was triturated with n-hexane and filtered to give 5-cyclopropyl-4-[3,4-difluoro-2-(methylsulphonyl)benzoyl]isoxazole (compound 1) (0.59g) as an orange solid, m.p.115-118°C.

By proceeding in a similar manner the following compounds of general formula (I) were prepared from the appropriately substituted starting materials.

| **Cpd No** | **R** | **R**^{**1**} | **R**^{**2**} | **R**^{**3**} | **R**^{**4**} | **m.p/NMR** |
|---|---|---|---|---|---|---|
| 2 | H | Cp | SMe | Cl | Cl | 83.5-84.5°C |
| 5 | H | Cp | SMe | OMe | Br | a |
| 6 | H | Cp | SO₂Me | OMe | Br | 146.4-146.8°C |
| 10 | H | Cp | SMe | OMe | Cl | b |
| 14 | H | Cp | SMe | Me | Cl | 85-87°C |
| 15 | H | Cp | SMe | F | Cl | 73-74°C |
| 20 | H | Cp | SMe | CO₂Me | CF₃ | c |
| 21 | H | Cp | SMe | CO₂Me | Cl | d |
| 22 | H | Cp | SMe | Cl | Br | 93-94°C |
| 27 | H | Cp | SMe | Cl | CF₃ | 89-90°C |
| 29 | H | Cp | SMe | F | Br | e |
| 32 | H | Cp | SMe | C(CH₃)=CH₂ | Cl | 140-141.5°C |
| 33 | H | Cp | SMe | Me | SMe | 103-105°C |
| Note: Cp = Cyclopropyl a = ¹H NMR (CDCl₃): 1.2(m,2H), 1.3(m,2H), 2.4(s,3H), 2.6(m,1H), 4.0(s,3H), 7.0(d,1H), 7.6(d,1H), 8.15(s,1H). b = ¹H NMR (CDCl₃): 1.2(m,2H) 1.4(m,2H), 2.4(s,3H), 2.6(m,1H), 4.0(s,3H), 7.05(d,1H), 7.45(d,1H), 8.15(s,1H). c = ¹H NMR (CDCl₃): 1.25(m,2H), 1.35(m,2H), 2.4(s,3H), 2.55(m,1H), 4.0(s,3H), 7.5(d,1H), 7.8(d,1H), 8.15(s,1H). d = ¹H NMR (CDCl₃): 1.2(m,2H), 1.35(m,2H), 2.4(s,3H), 2.5(m,1H), 4.0(s,3H), 7.35(d,1H), 7.55(d,1H), 8.15(s,1H). e = ¹H NMR (CDCl₃): 1.25 (m, 2H), 1.33(m,2H), 2.45(s,3H), 2.65(m,1H), 7.05(d,1H), 7.6(t,1H), 8.15(s,1H). | | | | | | |

### Example 2

A mixture of magnesium (0.17g) and methanol containing approximately 0.1 ml of carbon tetrachloride was heated at reflux for 0.5 hours, cooled and 3-cyclopropyl-1-[3,4-dichloro-2-(methylsulphenyl)phenyl]propane-1,3-dione (2.0g) was added. The mixture was stirred and heated at reflux for 2 hours. It was cooled and evaporated to dryness. The residue was dissolved in dichloromethane and a solution of ethyl chloro-oximidoacetate (1.37g) in dichloromethane was added. The mixture was stirred at room temperature overnight. Hydrochloric acid (2M) was added and the layers were separated. The organic layer was washed with water, dried (anhydrous Na₂SO₄) and filtered. The filtrate was evaporated to dryness and the residue was purified by dry column flash chromatography eluted with a mixture of ethyl acetate and n-hexane (1:9) to give ethyl 5-cyclopropyl-4-[3,4-dichloro-2-(methylsulphenyl) benzoyl]isoxazole-3-carboxylate (compound 8) (2.19g) as an orange oil NMR: (CDCl₃) 1.15-1.3(m,5H), 1.4(m,2H), 2.4(s,3H), 2.45(m,1H), 4.1(q,2H), 7.2(d,1H), 7.5(d,1H).

### Example 3

3-Chloroperoxybenzoic acid (2.0g) was added to a solution of 5-cyclopropyl-4-[3,4-dichloro-2-(methylsulphenyl)benzoyl]isoxazole ( 1.86g) in dichloromethane while maintaining the temperature around - 15°C. The mixture was stirred at -15°C for 1 hour and at room temperature for 1 hour. It was recooled to -15°C and filtered. The filtrate was evaporated to dryness and the residue was purified by dry column flash chromatography eluted with a mixture of ethyl acetate and n-hexane. The product was recrystallized from a mixture of ethyl acetate and n-hexane to give 5-cyclopropyl-4-[3,4-dichloro-2-(methylsulphinyl)benzoyl]isoxazole (compound 3) (0.3g) as a white solid, m.p. 110-112°C.

By proceeding in a similar manner the following compounds were prepared from the appropriately substituted starting materials:

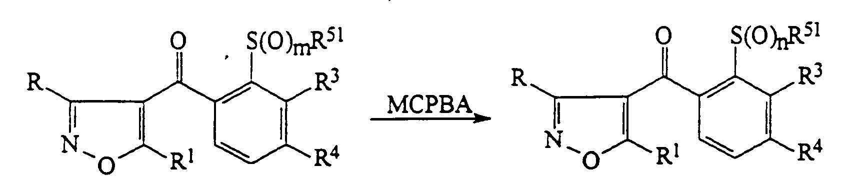

| **Cpd No** | **R** | **R**^{**1**} | **R**^{**51**} | **R**^{**3**} | **R**^{**4**} | **m** | **n** | **m.p (°C) /NMR** |
|---|---|---|---|---|---|---|---|---|
| 4 | H | Cp | Me | Cl | Cl | 1 | 2 | 149-150 |
| 7 | H | Cp | Me | OMe | Br | 0 | 1 | a |
| 9 | CO₂Et | Cp | Me | Cl | Cl | 0 | 1 | 129-130 |
| 11 | CO₂Et | Cp | Me | Cl | Cl | 1 | 2 | 106-107.5 |
| 12 | H | Cp | Me | OMe | Cl | 0 | 1 | 95-96 |
| 13 | H | Cp | Me | OMe | Cl | 0 | 2 | 63-67 |
| 16 | H | Cp | Me | F | Cl | 0 | 1 | 136-137 |
| 17 | H | Cp | Me | F | Cl | 0 | 2 | 151-152 |
| 18 | H | Cp | Me | Me | Cl | 0 | 1 | 115.4-118 |
| 19 | H | Cp | Me | Me | Cl | 0 | 2 | 132-134.6 |
| 23 | H | Cp | Me | Cl | Br | 0 | 1 | 133-134 |
| 24 | H | Cp | Me | Cl | Br | 0 | 2 | 147-148 |
| 25 | H | Cp | Me | CO₂Me | Cl | 0 | 1 | 163-164 |
| 26 | H | Cp | Me | CO₂Me | Cl | 0 | 2 | 123.4-132 |
| 28 | H | Cp | Me | Cl | CF₃ | 0 | 2 | 136-137 |
| 30 | H | Cp | Me | F | Br | 0 | 1 | 125-126 |
| 34 | H | Cp | Me | C(CH₃)=CH₂ | Cl | 0 | 1 | 215-217 |
| 35 | H | Cp | Me | C(CH₃)=CH₂ | Cl | 0 | 2 | 130-132 |
| Note: Cp = Cyclopropyl a = ¹H NMR (CDCl₃): 1.1-1.4(m,4H), 2.6(m,1H), 3.0(s,3H), 3.95(s,3H), 7.0(d,1H), 7.7(d,1H), 8.1(s,1H). | | | | | | | | |

### Example 4

Hydrogen peroxide (30%; 1.3 ml) was added dropwise to a solution of 4-[4-bromo-3-fluoro-2-(methylsulphenyl)benzoyl]-5-cyclopropylisoxazole (1.2 g) in a mixture of acetic acid and acetic anhydride. The resultant mixture was heated at 70°C for 4 hours. It was cooled, poured into water and extracted with ethyl acetate. The organic extract was washed with aqueous sodium bisulphite, aqueous ferrous sulphate and water, dried (MgSO₄) and filtered. The filtrate was evaporated to dryness and the residue was triturated with ether and altered to give 4-[4-bromo-3-fluoro-2-(methylsulphonyl)-benzoyl]-5-cyclopropylisoxazole (compound 31, 0.85 g) as a white solid, m.p. 144-145°C.

### Reference Example 1

A mixture of 3-cyclopropyl-1-[3,4-difluoro-2-(methylsulphonyl) phenyl]propane-1,3-dione (0.85g) and triethyl orthoformate (1.04g) in acetic anhydride was stirred and heated at reflux for 4 hours. It was evaporated to dryness and the residue was treated with toluene and re-evaporated to give 3-cyclopropyl-1-[3,4-difluoro-2-(methylsulphonyl)phenyl]-2-ethoxymethylenepropane-1,3-dione (1.13g) as a brown oil which was not purified further.

By proceeding in a similar manner the following compounds were prepared from the appropriately substituted starting materials;

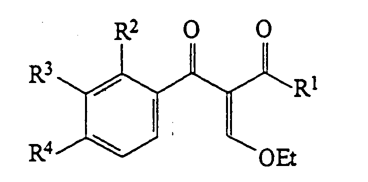

| **R**^{**1**} | **R**^{**2**} | **R**^{**3**} | **R**^{**4**} |
|---|---|---|---|
| Cyclopropyl | SMe | Cl | Cl |
| Cyclopropyl | SMe | OMe | Br |
| Cyclopropyl | SO₂Me | OMe | Br |
| Cyclopropyl | SMe | OMe | Cl |
| Cyclopropyl | SMe | Me | Cl |
| Cyclopropyl | SMe | F | Cl |
| Cyclopropyl | SMe | CO₂Me | CF₃ |
| Cyclopropyl | SMe | CO₂Me | Cl |
| Cyclopropyl | SMe | Cl | Br |
| Cyclopropyl | SMe | Cl | CF₃ |
| Cyclopropyl | SMe | F | Br |
| Cyclopropyl | SMe | C(CH₃)=CH₂ | Cl |
| Cyclopropyl | SMe | Me | SMe |

### Reference Example 2

Magnesium (0.17g) was suspended in methanol containing carbon tetrachloride (approximately 0.1 ml) and the mixture was warmed to initiate the reaction. t-Butyl 3-cyclopropyl-3-oxopropionate (1.32g) was added and the mixture was stirred for 1 hour. The mixture was evaporated to dryness and the residue was dissolved in toluene and re-evaporated. The residue was dissolved in acetonitrile and 3,4-difluoro-2-(methylsulphonyl)benzoyl chloride (1.83g) was added. The mixture was stirred at room temperature for 4 hours and left to stand overnight. The mixture was evaporated to dryness and the residue was partitioned between toluene and hydrochloric acid (2M). The layers were separated and the organic layer was washed with water then dried by azeotropic removal of water. 4-Toluene sulphonic acid (0.5g) was added to the mixture which was heated at reflux for 4 hours. After cooling it was washed with water, dried (anhydrous MgSO₄) and filtered. The filtrate was evaporated to dryness to give 3-cyclopropyl-1-[3,4-difluoro-2-(methylsulphonyl)phenyl]propane-1,3-dione (0.86g) as a brown solid which was not purified further.

By proceeding in a similar manner the following compounds were prepared from the appropriately substituted starting materials. In all cases the acetonitrile is replaced by toluene

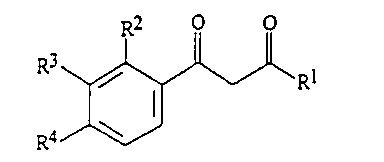

| **R**^{**1**} | **R**^{**2**} | **R**^{**3**} | **R**^{**4**} | **m.p/NMR** |
|---|---|---|---|---|
| Cp | SMe | Cl | Cl | 57-58.5°C |
| Cp | SMe | OMe | Br | a |
| Cp | SO₂Me | OMe | Br | - |
| Cp | SMe | OMe | Cl | b |
| Cp | SMe | F | Cl | Not purified further |
| Cp | SMe | CO₂Me | CF₃ | Not purified further |
| Cp | SMe | CO₂Me | Cl | Not purified further |
| Cp | SMe | Cl | Br | c |
| Cp | SMe | Cl | CF₃ | Not purified further |
| Cp | SMe | F | Br | d |
| Cp | SMe | C(CH₃)=CH₂ | Cl | 61-63°C |
| Note: Cp = Cyclopropyl a = NMR (CDCl₃): 0.9-1.4(m,4H), 1.7.1.9(m,1H), 2.5(s,3H), 3.95(s,3H), 5.9(s,1H), 7.0(d,1H), 7.4(d,1H). b = NMR (CDCl₃) 0.8-1.4(m,4H), 1.5-1.9(m,1H), 2.45(s,3H), 4.0(s,3H), 6.0(s,1H), 7.15(d,1H), 7.4(d,1H). c = NMR (CDCl₃): 1.0(m,2H), 1.2(m,2H), 1.75(m,1H), 2.5(s,3H), 5.95(s,1H), 7.2(d,1H), 7.65(d,1H) 15.7-16.1(bs,1H). d = NMR (CDCl₃): 1.0(m,2H), 1.25(m,2H), 1.75(m,1H), 2.5(s,3H), 6.0(s,1H), 7.2(d,1H) 7.55(dd,1H) 15.7-16.0(bs,1H). | | | | |

### Reference Example 3

A mixture of methyl 4-chloro-3-methyl-2-(methylsulphenyl)benzoate (19.5g) and cyclopropyl methyl ketone (13.4 g) in dry tetrahydrofuran was added to a stirred heated suspension of sodium hydride (80% oil dispersion, 4.8 g) in dry tetrahydrofuran. The mixture was stirred and heated at reflux for 2 hours. It was cooled and hydrochloric acid (2ml) was added. The layers were separated and the aqueous layer was extracted with ether. The combined organic layers were washed with water, saturated aqueous sodium bicarbonate, water, dried (Na₂SO₄) and altered. The filtrate was evaporated to dryness to give 1-[4-chloro-3-methyl-2-(methylsulphenyl)phenyl]-3-cyclopropylpropan-1,3-dione (20.19 g) as a yellow oil, NMR(CDCl₃) 0.9(m,2H), 1.2 (m,2H), 1.7(m,1H), 2.3(s,3H), 2.65(s,3H), 5,85 (s,1H), 7.15(d,1H), 7.3(d,1H), 15.7-16.0(bs,1H).

By proceeding in a similar manner the following compound was prepared from the appropriately substituted starting material;

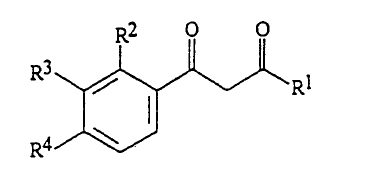

| **R**^{**1**} | **R**^{**2**} | **R**^{**3**} | **R**^{**4**} | **m.p/NMR** |
|---|---|---|---|---|
| Cp | SMe | Me | SMe | a |
| a = NMR (CDCl₃) 1.0(m,2H), 1.2(m,2H), 1.75(m,1H), 2.3(s,3H), 2.5(s,3H), 2.6(s,3H), 6.0(s,1H), 7.1(d,1H), 7.3(d,1H), 15.8-16.1(bs,1H) | | | | |

Benzoyl chlorides were prepared by heating the appropriately substituted benzoic acids at reflux with thionyl chloride for 3 hours. The excess thionyl chloride was removed by evaporation and the benzoyl chlorides were used directly without further purification.

### Reference Example 4

Hydrogen peroxide (11ml) was added with stirring to a cooled solution of 3,4-difluoro-2-(methylsulphenyl)benzoic acid (3.0g) and acetic anhydride (2.1ml) in acetic acid while maintaining the temperature below 5°C. The mixture was stirred at 0°C for 0.5 hours then warmed to room temperature. Further acetic acid was added and the mixture was stirred at room temperature for 0.5 hours and at 65°C for 2.5 hours. After cooling to room temperature water was added and the mixture was extracted with ethyl acetate, washed with water, aqueous ferrous sulphate solution and water, dried (anhydrous MgSO₄) and filtered. The filtrate was evaporated to dryness and the residue was recrystallised from a mixture of cyclohexane and ether to give 3,4-difluoro-2-(methylsulphonyl)benzoic acid (2.0g) as a white solid, m.p. 194°C.

### Reference Example 5

n-Butyllithium (2.5m in hexane, 35ml) was added with cooling to a solution of 3,4-difluorobenzoic acid (5.5g) in dry tetrahydrofuran while maintaining the temperature below -70°C. The mixture was stirred for 2 hours at -70°C. A solution of dimethyl disulphide (19.8g) in tetrahydrofuran was added and the mixture was stirred at -70°C for 1.5 hours. It was allowed to warm to room temperature, diluted with ether and washed with water. The aqueous layer was acidified to pH 1 and extracted with ether, washed with water, dried (anhydrous MgSO₄) and filtered. The filtrate was evaporated to dryness and the residue was recrystallised from a mixture of cyclohexane and ether to give 3,4-difluoro-2-(methylsulphenyl)benzoic acid (5.9g) as a white solid, m.p. 149.2-149.6°C.

By proceeding in a similar manner the following compounds were prepared from the appropriately substituted starting materials;

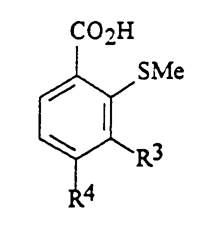

| **R**^{**3**} | **R**^{**4**} | **Reaction temp** | **m.p.** |
|---|---|---|---|
| F | Cl | -40°C | 145-146°C |
| Cl | CF₃ | -40°C | 97-100°C |

### Reference Example 6

A solution of sodium nitrite (4.53g) in water was added to a stirred suspension of 3,4-dichloroanthranillic acid (15g) in acetic acid and concentrated hydrochloric acid while maintaining the temperature below 5°C. The mixture was stirred at below 5°C for 2 hours then poured into a solution of dimethyl disulphide (8.4g) and copper powder (0.1g) in acetic acid. The mixture was stirred at room temperature for 1 hour and poured into water. The solid was filtered off, dried and recrystallized from cyclohexane to give 3,4-dichloro-2-(methylsulphenyl)benzoic acid (12.02g) as a pale yellow solid, NMR (DMSO - D₆) 2.4(s,3H), 7.5(d,1H), 7.7(d,1H), 13.5(bs,1H).

### Reference Example 7

A solution of potassium hydroxide (2.0g) in water was added to a solution of ethyl 4-bromo-3-methoxy-2-(methylsulphenyl)benzoate (4.5g) in ethanol. The resulting solution was stirred and heated at reflux for 3 hours. After cooling, the mixture was evaporated to dryness and the residue was dissolved in water and washed with ethyl acetate. The aqueous solution was acidified to pH 1 and extracted with ethyl acetate, dried (anhydrous MgSO₄) and filtered. The filtrate was evaporated to dryness to give 4-bromo-3-methoxy-2-(methylsulphenyl)benzoic acid as a white solid, NMR (CDCl₃) 2.5(s,3H), 3.9(s,3H), 7.4(s,2H), 10.9(bs,1H).

By proceeding in a similar manner the following compound was prepared from the appropriately substituted starting material:

4-Bromo-3-methoxy-2-(methylsulphonyl)benzoic acid NMR (CDCl₃) 3.3(s,3H), 4.1(s,3H), 7.1(d,1H), 7.75(d,1H), 8.2(bs,1H).

### Reference Example 8

A solution of methanethiol (47ml) in dimethyl formamide was added to a mixture of ethyl 2,4-dibromo-3-methoxybenzoate (105g) and potassium carbonate (131g) in dimethyl formamide and the resultant suspension was stirred at room temperature overnight. Water was added and the mixture was extracted into ether, washed with water, dried (MgSO₄) and altered. The filtrate was evaporated to dryness and the residue was purified by column chromatography eluted with a mixture of ethyl acetate and cyclohexane to give as the minor component ethyl 4-bromo-3-methoxy-2-(methylsulphenyl)benzoate (4.5g) as a white solid, NMR (CDCl₃) 1.5(t,3H), 2.6(s,3H), 4.05(s,3H), 4.5(q,2H), 7.35 (m,2H).

### Reference Example 9

Hydrogen peroxide (11.3ml) was added to a cooled solution of ethyl 4-bromo-3-methoxy-2-(methylsulphenyl)benzoate (3.7g) and acetic anhydride (2.0ml) in acetic acid at 0°C. The mixture was stirred at 0°C for 1 hour then warmed to room temperature and heated at 85°C for 3 hours. After cooling to room temperature the mixture was diluted with ethyl acetate and washed with water, aqueous ferrous sulphate solution, water, dried (MgSO₄) and altered. The filtrate was evaporated to dryness to give ethyl 4-bromo-3-methoxy-2-(methylsulphonyl)benzoate (3.6g) as a yellow oil, NMR (CDCl₃) 1.6(t,3H), 3.5(s,3H), 4.3(s,3H), 4.55(q,2H), 7.2(d,1H), 7.95(d,1H).

### Reference Example 10

A mixture of 2-[4-chloro-3-methoxy-2-(methylsulphenyl)phenyl]-4,4-dimethyloxazoline (9.0g) and hydrochloric acid (5M) was stirred and heated at reflux for 5 hours. After cooling, the mixture was diluted with water and extracted with dichloromethane. It was dried (MgSO₄) filtered and the filtrate was evaporated to dryness to give 4-chloro-3-methoxy-2-(methylsulphenyl) benzoic acid as a white solid, m.p. 98-99°C.

### Reference Example 11

n-Butyllithium (2.5M in hexane, 54ml) was added with cooling to a stirred solution of 2-(4-chloro-3-methoxyphenyl)-4,4-dimethyloxazoline (27.0g) in tetrahydrofuran while maintaining the temperature below -40°C. The mixture was stirred at -78°C overnight. A solution of dimethyl disulphide (26.5g) in tetrahydrofuran was added dropwise and the mixture was stirred at -40°C overnight. After allowing to warm to room temperature the mixture was treated with hydrochloric acid (2M). The organic layer was washed with water, dried (MgSO₄) and filtered. The filtrate was evaporated to dryness and the residue was purified by dry column flash chromatography eluted with a mixture of ethyl acetate and n-hexane to give 2-[4-chloro-3-methoxy-2-(methylsulphenyl) phenyl]-4,4-dimethyloxazoline (11.1g) as a white solid, m.p. 50-52°C.

### Reference Example 12

n-Butyllithium (2.5M in hexane, 63ml) was added to a solution of diisopropylamine in dry tetrahydrofuran while maintaining the temperature at 0°C. Once addition was complete the cooling bath was removed and the mixture stirred for 30 minutes at room temperature. The resulting solution of lithium di-isopropylamide (LDA) was then added to a solution of 4-bromo-3-fluorobenzoic acid (14.6g) in tetrahydrofuran while maintaining the temperature at -50°C. The mixture was then stirred for 5 hours at -30°C. A solution of dimethyl disulphide (21g) in tetrahydrofuran was then added and the cooling bath was removed and the mixture allowed to stir at room temperature overnight. The mixture was diluted with ether and washed with water. The aqueous layer was acidified to pH 1 with 2M hydrochloric acid and extracted with ether, washed with water, dried (MgSO₄) and filtered. The filtrate was evaporated to dryness and the residue triturated with petroleum spirit (b.p. 60-80°C) to give 4-bromo-3-fluoro-2-(methylsulphenyl)benzoic acid (14g) as a white solid, m.p. 152-154°C.

By proceeding in a similar manner from the appropriately substituted starting material 4-bromo-3-chloro-2-(methylsulphenyl)benzoic acid was prepared, m.p. 126-129°C.

### Reference Example 13

A solution of 4-bromo-3-fluorotoluene (35g) and sodium hydroxide (7.7g) in pyridine and water was stirred and heated to reflux. Potassium permanganate (123g) was added to the mixture over 2 hours. The resulting suspension was heated at reflux for a further 3 hours. The mixture was filtered hot through hyflo. "Hyflo" is a Trade Mark. The hyflo was washed with boiling water, followed by ethyl acetate. The cooled aqueous layer was acidified to pH 1 with concentrated hydrochloric acid and extracted with ethyl acetate. The organic extract was washed with water, dried (MgSO₄) and filtered. The filtrate was evaporated to dryness and the residue triturated with petroleum spirit (bp 60-80°C) to give 4-bromo-3-fluorobenzoic acid as a white solid (21.25g), m.p. 213-215°C.

### Reference Example 14

Lithium hydroxide monohydrate (1.87 g) was added to a solution of methyl 3-methoxycarbonyl-2-(methylsulphenyl)-4-trifluoromethylbenzoate (13.71 g) in methanol and water. The mixture was stirred at room temperature overnight and the methanol was removed by evaporation. The residual aqueous solution was acidified to pH 1 and extracted with ether, washed with water, dried (MgSO₄) and filtered. The filtrate was evaporated to dryness to give 3-methoxycarbonyl-2-(methylsulphenyl)-4-trifluoromethylbenzoic acid (10.85 g) as an off-white solid, NMR (CDCl₃) 2.45(s,3H), 3.95(s,3H), 5.45-6.1(bs,1H), 7.2(d,1H), 7.95(d,1H).

By proceeding in a similar manner the following compound was prepared from the appropriately substituted starting material:

4-chloro-3-methoxycarbonyl-2-(methylsulphenyl)benzoic acid, NMR (CDCl₃) 2.5 (s,3H), 4.0 (s,3H), 7.55(d,1H), 8.0(d,1H).

### Reference Example 15

Sodium thiomethoxide (6.83 g) was added to a solution of methyl 2-fluoro-3-methoxycarbonyl-4-trifluoromethylbenzoate (24.85 g) in xylene. After stirring for 0.5 hours lithium hydroxide monohydrate (4.10 g) was added and the mixture was stirred for 48 hours. Hydrochloric acid (2M) was added. It was extracted with ether, washed with water, dried (MgSO₄) and filtered. The filtrate was evaporated to dryness and the residue was triturated with cyclohexane. The solid was filtered off and the filtrate was evaporated to dryness to give methyl 3-methoxycarbonyl-2-(methylsulphenyl)-4-trifluoromethylbenzoate (13.71g) as a yellow oil NMR (CDCl₃) 2.4(s,3H), 3.95 (s,6H), 7.65 (s,2H).

By proceeding in a similar manner methyl 4-chloro-3-methoxycarbonyl-2-(methylsulphenyl)benzoate was prepared from the appropriately substituted starting material.

### Reference Example 16

A solution of 2-fluoro-3-methoxycarbonyl-4-trifluoromethylbenzoic acid (23.43 g) in thionyl chloride was stirred and heated at reflux for 2 hours, cooled and evaporated to dryness. The residue was dissolved in methanol and the resultant solution was stirred and heated at reflux overnight. It was cooled and evaporated to dryness. The residue was purified by chromatography eluted with a mixture of ether and cyclohexane to give methyl 2-fluoro-3-methoxycarbonyl-4-trifluoromethylbenzoate (25.85 g) as a yellow oil, NMR (CDCl₃) 4.0(s,6H), 7.55(d,1H), 8. 15(t,1H).

By proceeding in a similar manner the following compounds were prepared from the appropriately substituted starting material:
methyl 4-chloro-2-fluoro-3-methoxycarbonylbenzoate NMR (CDCl₃) 3.9(s,3H), 4.0(s,3H), 7.3(d,1H), 7.95(t,1H);
methyl 4-chloro-2-fluoro-3-methylbenzoate NMR (CDCl₃) 2.35(d,3H), 3.95(s,3H), 7.2(d,1H) 7.7(t.1H);
methyl 4-chloro-2-fluoro-3-isopropenylbenzoate NMR (CDCl₃) 2.1(s,3H), 3.95(s,3H), 5.0(s,1H) 5.45(s,1H) 7.3(d,1H) 7.8(t,1H).

### Reference Example 17

A solution of lithium diisopropyl amide in dry tetrahydrofuran (prepared from diisopropylamine (17.0 ml) and n-butylithium (48.4 ml) in dry tetrahydrofuran)was added to a solution of methyl 2-fluoro-6-trifluoromethylbenzoate (22.39 g) in dry tetrahydrofuran while maintaining the temperature below -70°C. The mixture was stirred at -78°C for 3 hours. The solution was poured onto solid carbon dioxide pellets and stirred until it had warmed to room temperature. The mixture was evaporated and treated with hydrochloric acid (2M). It was extracted with ether, washed with water, dried (MgSO₄) and altered. The filtrate was evaporated to dryness to give 2-fluoro-3-methoxycarbonyl-4-trifluoromethylbenzoic acid (24.43 g) as an off-white solid NMR (CDCl₃) 3.95(s,3H), 7.15(d,1H), 8. 15(t,1H).

By proceeding in a similar manner the following compound was prepared from the appropriately substituted starting material:
4-chloro-2-fluoro-3-methoxycarbonylbenzoic acid NMR (CDCl₃) 4.0(s,3H), 7.4(d,1H), 8.1(t,1H).

### Reference Example 18

Potassium carbonate (48.37 g) was added to a solution of methanethiol (16.84 g) in dry dimethyl formamide. Methyl 4-chloro-2-fluoro-3-methylbenzoate (35.45 g) was added to the resulting suspension. The mixture was stirred for 60 hours. It was poured into water, extracted with ether, washed with water, dried (Na₂SO₄) and filtered. The filtrate was evaporated to dryness and the residue was separated by chromatography eluted with a mixture of ether and hexane to give methyl 4-chloro-3-methyl-2-(methylsulphenyl)benzoate (19.53 g) as a clear oil, NMR (CDCl₃) 2.35(s,3H), 2.7(s,3H), 3.95(s,3H), 7.25(d,1H), 7.4(d,1H), and methyl 3-methyl-2,4-bis(methylsulphenyl)benzoate (9.29 g) as a yellow solid NMR (CDCl₃) 2.3 (s,3H), 2.5 (s,3H), 2.6 (s,3H), 3.95 (s,3H), 7.1 (d,1H), 7.4 (d,1H).

### Reference Example 19

n-Butyllithium (2.5 M in hexane, 100 ml) was added to a cooled solution of 2-chloro-6-fluorotoluene (36.1 g) in dry tetrahydrofuran while maintaining the temperature below -60°C. The mixture was stirred at -78°C overnight then poured onto solid carbon dioxide pellets. The mixture was stirred and allowed to warm to room temperature. It was acidified to pH 1 and extracted with ether. The organic layer was extracted into aqueous sodium hydroxide solution (2M) and water. The combined aqueous extracts were acidified to pH 1 and the solid formed was filtered off and washed with water and n-hexane to give 4-chloro-2-fluoro-3-methylbenzoic acid (40.35 g) as a white solid, NMR (DMSO-d₆) 2.3(d,3H), 7.4(d,1H) 7.7(t,1H).

By proceeding in a similar manner the following compound was prepared from the appropriately substituted starting material:
4-chloro-2-fluoro-3-isopropenylbenzoic acid m.p. 201-202°C.

### Reference Example 20

A solution of sodium hydroxide (7.0 g) in water was added to methyl 4-chloro-3-isopropenyl-2-(methylsulphenyl)benzoate (7.3 g) and the resulting mixture was heated at reflux for 2 hours. Ethanol was added and the mixture was heated at reflux for 1 hour. The ethanol was removed by evaporation and the aqueous residue was acidified to pH 1. It was extracted with ethyl acetate, washed with water, dried (MgSO₄) and filtered. The titrate was evaporated to dryness to give 4-chloro-3-isopropenyl-2-(methylsulphenyl)benzoic acid (6.15 g) NMR (CDCl₃) 2.05 (s,3H), 2.4 (s,3H), 4.85 (s,1H), 5.35 (s,1H), 7.45 (d,1H), 7.85 (d,1H).

### Reference Example 21

A mixture of methyl 4-chloro-2-fluoro-3-isopropenylbenzoate (8.6 g) and sodium thiomethoxide (3.15 g) in dimethyl formamide was heated at 50°C for 3 hours and stirred at room temperature over-night. Ether was added and the mixture was washed with water, dried (MgSO₄) and filtered. The filtrate was evaporated to dryness and the residue was purified by chromatography eluted with a mixture of ethyl acetate and hexane to give methyl 4-chloro-3-isopropenyl-2-(methylsulphenyl)benzoate (5.95 g) as a clear oil NMR (CDCl₃) 2.1(s,3H), 2.35(s,3H), 3.95(s,3H), 4.9(s,1H), 5.4(s,1H), 7.45(s,2H).

### Reference Example 22

A mixture of 2-(2-chloro-6-fluorophenyl)propan-2-ol (19.0g), concentrated sulphuric acid and water was heated at reflux for 2 hours. It was cooled and extracted with ether, washed with water, dried (MgSO₄) and filtered. The filtrate was evaporated to dryness and the residue was purified by chromatography eluted with hexane to give 2-(2-chloro-6-fluorophenyl)propene (11.6 g) as a clear oil, NMR (CDCl₃) 1.95(s,3H) 4.95(s,1H), 5.85(s,1H), 6.85-7.0(m,1H), 7.05-7.2(m,2H).

### Reference Example 23

A solution of methyl 2-chloro-6-fluorobenzoate (40.0g) in ether was added to a solution of methyl magnesium iodide in ether (prepared from methyl iodide (120.0g) and magnesium turnings (20.6g) in ether). The resulting solution was stirred and heated at reflux for 5 hours, poured onto a mixture of ice and concentrated sulphuric acid and the layers separated. The organic layer was washed with water, saturated aqueous sodium bisulphite, water, dried (anhydrous magnesium sulphate) and filtered. The filtrate was evaporated to dryness to give 2-(2-chloro-6-fluorophenyl)propan-2-ol (36.8g) as an orange oil, NMR (CDCl₃) 1.8(d,6H), 3.5-3.7(bs,1H), 6.9-7.05(m,1H), 7.1-7.25(m,2H).

According to a feature of the present invention, there is provided a method for controlling the growth of weeds (i.e. undesired vegetation) at a locus which comprises applying to the locus a herbicidally effective amount of at least one isoxazole derivative of general formula (I). For this purpose, the isoxazole derivatives are normally used in the form of herbicidal compositions (i.e. in association with compatible diluents or carriers and/or surface active agents suitable for use in herbicidal compositions), for example as hereinafter described.

The compounds of general formula (I) show herbicidal activity against dicotyledonous (i.e. broad-leafed) and monocotyledonous (i.e. grass) weeds by pre- and/or post-emergence application.

By the term "pre-emergence application" is meant application to the soil in which the weed seeds or seedlings are present before emergence of the weeds above the surface of the soil. By the term "post-emergence application" is meant application to the aerial or exposed portions of the weeds which have emerged above the surface of the soil. For example, the compounds of general formula (I) may be used to control the growth of:
broad-leafed weeds, for example, Abutilon theophrasti, Amaranthus retroflexus, Bidens pilosa, Chenopodium album, Galium aparine, Ipomoea spp. e.g. Ipomoea purpurea, Sesbania exaltata, Sinapis arvensis, Solanum nigrum and Xanthium strumarium, and
grass weeds, for example Alopecurus myosuroides, Avena fatua, Digitaria sanguinalis, Echinochloa crus-galli, Sorghum bicolor, Eleusine indica and Setaria spp, e.g. Setaria faberii or Setaria viridis, and
sedges, for example, Cyperus esculentus.

The amounts of compounds of general formula (I) applied vary with the nature of the weeds, the compositions used, the time of application, the climatic and edaphic conditions and (when used to control the growth of weeds in crop-growing areas) the nature of the crops. When applied to a crop-growing area, the rate of application should be sufficient to control the growth of weeds without causing substantial permanent damage to the crop. In general, taking these factors into account, application rates between 0.01kg and 5kg of active material per hectare give good results. However, it is to be understood that higher or lower application rates may be used, depending upon the particular problem of weed control encountered.

The compounds of general formula (I) may be used to control selectively the growth of weeds, for example to control the growth of those species hereinbefore mentioned, by pre- or post-emergence application in a directional or non-directional fashion, e.g. by directional or non-directional spraying, to a locus of weed infestation which is an area used, or to be used, for growing crops, for example cereals, e.g. wheat, barley, oats, maize and rice, soya beans, field and dwarf beans, peas, lucerne, cotton, peanuts, flax onions, carrots, cabbage, oilseed rape, sunflower, sugar beet, and permanent or sown grassland before or after sowing of the crop or before or after emergence of the crop. For the selective control of weeds at a locus of weed infestation which is an area used, or to be used, for growing of crops, e.g. the crops hereinbefore mentioned, application rates between 0.01kg and 4.0kg, and preferably between 0.01kg and 2.0kg, of active material per hectare are particularly suitable.

The compounds of general formula (I) may also be used to control the growth of weeds, especially those indicated above, by pre- or post-emergence application in established orchards and other tree-growing areas, for example forests, woods and parks, and plantations, e.g. sugar cane, oil palm and rubber plantations. For this purpose they may be applied in a directional or non-directional fashion (e.g. by directional or non-directional spraying) to the weeds or to the soil in which they are expected to appear, before or after planting of the trees or plantations at application rates between 0.25kg and 5.0kg, and preferably between 0.5kg and 4.0kg of active material per hectare.

The compounds of general formula (I) may also be used to control the growth of weeds, especially those indicated above, at loci which are not crop-growing areas but in which the control of weeds is nevertheless desirable.

Examples of such non-crop-growing areas include airfields, industrial sites, railways, roadside verges, the verges of rivers, irrigation and other waterways, scrublands and fallow or uncultivated land, in particular where it is desired to control the growth of weeds in order to reduce fire risks. When used for such purposes in which a total herbicidal effect is frequently desired, the active compounds are normally applied at dosage rates higher than those used in crop-growing areas as hereinbefore described. The precise dosage will depend upon the nature of the vegetation treated and the effect sought.

Pre- or post-emergence application, and preferably pre-emergence application, in a directional or non-directional fashion (e.g. by directional or non-directional spraying) at application rates between 1.0kg and 20.0kg, and preferably between 5.0 and 10.0kg, of active material per hectare are particularly suitable for this purpose.

When used to control the growth of weeds by pre-emergence application, the compounds of general formula (I) may be incorporated into the soil in which the weeds are expected to emerge. It will be appreciated that when the compounds of general formula (I) are used to control the growth of weeds by post-emergence application, i.e. by application to the aerial or exposed portions of emerged weeds, the compounds of general formula (I) will also normally come into contact with the soil and may also then exercise a pre-emergence control on later-germinating weeds in the soil.

Where especially prolonged weed control is required, the application of the compounds of general formula (I) may be repeated if required.

According to a further feature of the present invention, there are provided compositions suitable for herbicidal use comprising one or more of the isoxazole derivatives of general formula (I), in association with, and preferably homogeneously dispersed in, one or more compatible agriculturally- acceptable diluents or carriers and/or surface active agents [i.e. diluents or carriers and/or surface active agents of the type generally accepted in the art as being suitable for use in herbicidal compositions and which are compatible with compounds of general formula (I)]. The term "homogeneously dispersed" is used to include compositions in which the compounds of general formula (I) are dissolved in other components. The term "herbicidal compositions" is used in a broad sense to include not only compositions which are ready for use as herbicides but also concentrates which must be diluted before use. Preferably, the compositions contain from 0.05 to 90% by weight of one or more compounds of general formula (I).

The herbicidal compositions may contain both a diluent or carrier and surface-active (e.g. wetting, dispersing, or emulsifying) agent. Surface-active agents which may be present in herbicidal compositions of the present invention may be of the ionic or non-ionic types, for example sulphoricinoleates, quaternary ammonium derivatives, products based on condensates of ethylene oxide with alkyl and polyaryl phenols, e.g. nonyl-or octyl-phenols, or carboxylic acid esters of anhydrosorbitols which have been rendered soluble by etherification of the free hydroxy groups by condensation with ethylene oxide, alkali and alkaline earth metal salts of sulphuric acid esters and sulphonic acids such as dinonyl- and dioctyl-sodium sulphonosuccinates and alkali and alkaline earth metal salts of high molecular weight sulphonic acid derivatives such as sodium and calcium lignosulphonates and sodium and calcium alkylbenzene sulphonates.

Suitably, the herbicidal compositions according to the present invention may comprise up to 10% by weight, e.g. from 0.05% to 10% by weight, of surface-active agent but, if desired; herbicidal compositions according to the present invention may comprise higher proportions of surface-active agent, for example up to 15% by weight in liquid emulsifiable suspension concentrates and up to 25% by weight in liquid water soluble concentrates.

Examples of suitable solid diluents or carriers are aluminium silicate, talc, calcined magnesia, kieselguhr, tricalcium phosphate, powdered cork, adsorbent carbon black and clays such as kaolin and bentonite. The solid compositions (which may take the form of dusts, granules or wettable powders) are preferably prepared by grinding the compounds of general formula (I) with solid diluents or by impregnating the solid diluents or carriers with solutions of the compounds of general formula (I) in volatile solvents, evaporating the solvents and, if necessary, grinding the products so as to obtain powders. Granular formulations may be prepared by absorbing the compounds of general formula (I) (dissolved in suitable solvents, which may, if desired, be volatile) onto the solid diluents or carriers in granular form and, if desired, evaporating the solvents, or by granulating compositions in powder form obtained as described above. Solid herbicidal compositions, particularly wettable powders and granules, may contain wetting or dispersing agents (for example of the types described above), which may also, when solid, serve as diluents or carriers.

Liquid compositions according to the invention may take the form of aqueous, organic or aqueous-organic solutions, suspensions and emulsions which may incorporate a surface-active agent. Suitable liquid diluents for incorporation in the liquid compositions include water, glycols, tetrahydrofurfuryl alcohol, acetophenone, cyclohexanone, isophorone, toluene, xylene, mineral, animal and vegetable oils and light aromatic and naphthenic fractions of petroleum (and mixtures of these diluents). Surface-active agents, which may be present in the liquid compositions, may be ionic or non-ionic (for example of the types described above) and may, when liquid, also serve as diluents or carriers.

Powders, dispersible granules and liquid compositions in the form of concentrates may be diluted with water or other suitable diluents, for example mineral or vegetable oils, particularly in the case of liquid concentrates in which the diluent or carrier is an oil, to give compositions ready for use.

When desired, liquid compositions of the compound of general formula (I) may be used in the form of self-emulsifying concentrates containing the active substances dissolved in the emulsifying agents or in solvents containing emulsifying agents compatible with the active substances, the simple addition of water to such concentrates producing compositions ready for use.

Liquid concentrates in which the diluent or carrier is an oil may be used without further dilution using the electrostatic spray technique.

Herbicidal compositions according to the present invention may also contain, if desired, conventional adjuvants such as adhesives, protective colloids, thickeners, penetrating agents, stabilisers, sequestering agents, anti-caking agents, colouring agents and corrosion inhibitors. These adjuvants may also serve as carriers or diluents.

Unless otherwise specified, the following percentages are by weight. Preferred herbicidal compositions according to the present invention are
aqueous suspension concentrates which comprise from 10 to 70% of one or more compounds of general formula (I), from 2 to 10% of surface-active agent, from 0.1 to 5% of thickener and from 15 to 87.9% of water;
wettable powders which comprise from 10 to 90% of one or more compounds of general formula (I), from 2 to 10% of surface-active agent and from 8 to 88% of solid diluent or carrier;
water soluble or water dispersible powders which comprise from 10 to 90% of one or more compounds of general formula (I), from 2 to 40% of sodium carbonate and from 0 to 88% of solid diluent;
liquid water soluble concentrates which comprise from 5 to 50%, e.g. 10 to 30%, of one or more compounds of general formula (I), from 5 to 25% of surface-active agent and from 25 to 90%, e.g. 45 to 85%, of water miscible solvent, e.g. dimethylformamide, or a mixture of water-miscible solvent and water;
liquid emulsifiable suspension concentrates which comprise from 10 to 70% of one or more compounds of general formula (I), from 5 to 15% of surface-active agent, from 0.1 to 5% of thickener and from 10 to 84.9% of organic solvent;
granules which comprise from 1 to 90%, e.g. 2 to 10% of one or more compounds of general formula (I), from 0.5 to 7%, e.g. 0.5 to 2%, of surface-active agent and from 3 to 98.5%, e.g. 88 to 97.5%, of granular carrier and
emulsifiable concentrates which comprise 0.05 to 90%, and preferably from 1 to 60% of one or more compounds of general formula (I), from 0.01 to 10%, and preferably from 1 to 10%, of surface-active agent and from 9.99 to 99.94%, and preferably from 39 to 98.99%, of organic solvent.

Herbicidal compositions according to the present invention may also comprise the compounds of general formula (I) in association with, and preferably homogeneously dispersed in, one or more other pesticidally active compounds and, if desired, one or more compatible pesticidally acceptable diluents or carriers, surface-active agents and conventional adjuvants as hereinbefore described. Examples of other pesticidally active compounds which may be included in, or used in conjunction with, the herbicidal compositions of the present invention include herbicides, for example to increase the range of weed species controlled for example alachlor [2-chloro-2,6'-diethyl-N-(methoxy-methyl)-acetanilide], atrazine [2-chloro-4-ethylamino-6-isopropylamino-1,3,5-triazine], bromoxynil [3,5-dibromo-4-hydroxybenzonitrile], chlortoluron [N'-(3-chloro-4-methylphenyl)-N,N-dimethylurea], cyanazine [2-chloro-4-(1-cyano-1-methylethylamino)-6-ethylamino-1,3,5-triazine], 2,4-D [2,4-dichlorophenoxy-acetic acid], dicamba [3,6-dichloro-2-methoxybenzoic acid], difenzoquat [1,2- dimethyl-3,5-diphenylpyrazolium salts], flampropmethyl [methyl N-2-(N- benzoyl-3-chloro-4-fluoroanilino)-propionate], fluometuron [N'-(3-trifluoro- methylphenyl)-N,N-dimethylurea], isoproturon [N'-(4-isopropylphenyl)-N,N-dimethylurea], insecticides, e.g. synthetic pyrethroids, e.g. permethrin and cypermethrin, and fungicides, e.g. carbamates, e.g. methyl N-(1-butyl-carbamoyl- benzimidazol-2-yl)carbamate, and triazoles e.g. 1-(4-chloro-phenoxy)-3,3- dimethyl-1-(1,2,4-triazol-1-yl)-butan-2-one.

Pesticidally active compounds and other biologically active materials which may be included in, or used in conjunction with, the herbicidal compositions of the present invention, for example those hereinbefore mentioned, and which are acids, may, if desired, be utilized in the form of conventional derivatives,.for example alkali metal and amine salts and esters.

According to a further feature of the present invention there is provided an article of manufacture comprising at least one of the isoxazole derivatives of general formula (I) or, as is preferred, a herbicidal composition as hereinbefore described, and preferably a herbicidal concentrate which must be diluted before use, comprising at least one of the isoxazole derivatives of general formula (I) within a container for the aforesaid derivative or derivatives of general formula (I), or a said herbicidal composition, and instructions physically associated with the aforesaid container setting out the manner in which the aforesaid derivative or derivatives of general formula (I) or herbicidal composition contained therein is to be used to control the growth of weeds. The containers will normally be of the types conventionally used for the storage of chemical substances which are solid at normal ambient temperatures and herbicidal compositions particularly in the form of concentrates, for example cans and drums of metal, which may be internally lacquered, and plastics materials, bottles or glass and plastics materials and, when the contents of the container is a solid, for example granular, herbicidal compositions, boxes, for example of cardboard, plastics materials and metal, or sacks. The containers will normally be of sufficient capacity to contain amounts of the isoxazole derivative or herbicidal compositions sufficient to treat at least one acre of ground to control the growth of weeds therein but will not exceed a size which is convenient for conventional methods of handling. The instructions will be physically associated with the container, for example by being printed directly thereon or on a label or tag affixed thereto. The directions will normally indicate that the contents of the container, after dilution if necessary, are to be applied to control the growth of weeds at rates of application between 0.01kg and 20kg of active material per hectare in the manner and for the purposes hereinbefore described.

The following Examples illustrate herbicidal compositions according to the present invention:

### EXAMPLE C1

A soluble concentrate is formed from

| | |
|---|---|
| Active ingredient (compound 1) | 20% w/v |
| Potassium hydroxide solution 33% w/v | 10% v/v |
| Tetrahydrofurfuryl alcohol (THFA) | 10% v/v |
| Water | to 100 volumes. |

by stirring THFA, active ingredient (compound 1) and 90% volume of water and slowly adding the potassium hydroxide solution until a steady pH 7-8 is obtained then making up to volume with water.

Similar soluble concentrates may be prepared as described above by replacing the isoxazole (compound 1) with other compounds of general formula (I).

### EXAMPLE C2

A wettable powder is formed from:

| | |
|---|---|
| Active ingredient (compound 1) | 50% w/w |
| Sodium dodecylbenzene sulphonate | 3% w/w |
| Sodium lignosulphate | 5% w/w |
| Sodium formaldehyde alkylnaphthalene sulphonate | 2% w/w |
| Microfine silicon dioxide | 3% w/w and |
| China clay | 37% w/w |

by blending the above ingredients together and grinding the mixture in an air jet mill.

Similar wettable powders may be prepared as described above by replacing the isoxazole (compound 1) with other compounds of general formula (I).

### EXAMPLE C3

A water soluble powder is formed from:

| | |
|---|---|
| Active ingredient (compound 1) | 50% w/w |
| Sodium dodecylbenzenesulphonate | 1% w/w |
| Microfine silicon dioxide | 2% w/w |
| Sodium bicarbonate | 47% w/w |

by mixing the above ingredients and grinding the above mixture in a hammer mill.

Similar water soluble powders may be prepared as described above by replacing the isoxazole (compound 1) with other compounds of general formula (I).

The compounds of the invention have been used in herbicidal applications according to the following procedures.

### METHOD OF USE OF HERBICIDAL COMPOUNDS:

### a) General

Appropriate quantities of the compounds used to treat the plants were dissolved in acetone to give solutions equivalent to application rates of up to 4000g test compound per hectare (g/ha). These solutions were applied from a standard laboratory herbicide sprayer delivering the equivalent of 290 litres of spray fluid per hectare.

### b) Weed control : Pre-emergence

The seeds were sown in 70 mm square, 75 mm deep plastic pots in non-sterile soil. The quantities of seed per pot were as follows:-

| Weed species | Approx number of seeds/pot |
|---|---|
| 1) Broad-leafed weeds | |
| Abutilon theophrasti | 10 |
| Amaranthus retroflexus | 20 |
| Galium aparine | 10 |
| Ipomoea purpurea | 10 |
| Sinapis arvensis | 15 |
| Xanthium strumarium | 2. |

| 2) Grass weeds | |
|---|---|
| Alopecurus myosuroides | 15 |
| Avena fatua | 10 |
| Echinochloa crus-galli | 15 |
| Setaria viridis | 20. |

| 3) Sedges | |
|---|---|
| Cyperus esculentus | 3. |

| Crop | |
|---|---|
| 1) Broad-leafed | |
| Cotton | 3 |
| Soya | 3. |

| 2) Grass | |
|---|---|
| Maize | 2 |
| Rice | 6 |
| Wheat | 6. |

The compounds of the invention were applied to the soil surface, containing the seeds, as described in (a). A single pot of each crop and each weed was allocated to each treatment, with unsprayed controls and controls sprayed with acetone alone.

After treatment the pots were placed on capillary matting kept in a glass house, and watered overhead. Visual assessment of crop damage was made 20-24 days after spraying. The results were expressed as the percentage reduction in growth or damage to the crop or weeds, in comparison with the plants in the control pots.

### c) Weed control : Post-emergence

The weeds and crops were sown directly into John Innes potting compost in 75 mm deep, 70 mm square pots except for Amaranthus which was pricked out at the seedling stage and transferred to the pots one week before spraying. The plants were then grown in the greenhouse until ready for spraying with the compounds used to treat the plants. The number of plants per pot were as follows :-
1) Broad leafed weeds

| Weed species | Number of plants per pot | Growth stage |
|---|---|---|
| Abutilon theophrasti | 3 | 1-2 leaves |
| Amaranthus retroflexus | 4 | 1-2 leaves |
| Galium aparine | 3 | 1^{st} whorl |
| Ipomoea purpurea | 3 | 1-2 leaves |
| Sinapis arvensis | 4 | 2 leaves |
| Xanthium strumarium | 1 | 2-3 leaves. |

2) Grass weeds

| Weed species | Number of plants per pot | Growth stage |
|---|---|---|
| Alopecurus myosuroides | 8-12 | 1-2 leaves |
| Avena fatua | 12-18 | 1-2 leaves |
| Echinochloa crus-galli | 4 | 2-3 leaves |
| Setaria viridis | 15-25 | 1-2 leaves. |

3) Sedges

| Weed species | Number of plants per pot | Growth stage |
|---|---|---|
| Cyperus esculentus | 3 | 3 leaves. |

1) Broad leafed

| Crops | Number of plants per pot | Growth stage |
|---|---|---|
| Cotton | 2 | 1 leaf |
| Soya | 2 | 2 leaves. |

2) Grass

| Crops | Number of plants per pot | Growth stage |
|---|---|---|
| Maize | 2 | 2-3 leaves |
| Rice | 4 | 2-3 leaves |
| Wheat | 5 | 2-3 leaves. |

The compounds used to treat the plants were applied to the plants as described in (a). A single pot of each crop and weed species was allocated to each treatment, with unsprayed controls and controls sprayed with acetone alone.

After treatment the pots were placed on capillary matting in a glass house, and watered overhead once after 24 hours and then by controlled sub-irrigation. Visual assessment of crop damage and weed control was made 20-24 days after spraying. The results were expressed as the percentage reduction in growth or damage to the crop or weeds, in comparison with the plants in the control pots.

The compounds of the invention have shown an excellent level of herbicidal activity together with crop tolerance on the weeds used in the foregoing experiments.

When applied pre- or post-emergence at 1000g/ha compounds 1 to 35 gave at least 90% reduction in growth of one or more of the weed species.

## Claims

1. A 4-benzoyl isoxazole derivative of general formula (I) 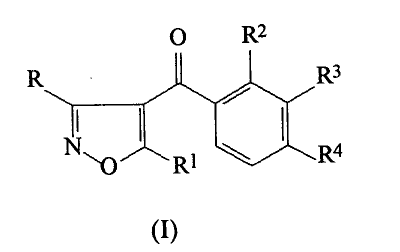 wherein:
R represents a hydrogen atom or a group -CO₂R⁵;
R¹ represents cyclopropyl;
R² represents -S(O)ₙR⁵¹;
R³ represents:
a chlorine, bromine or fluorine atom;
a straight- or branched- chain alkyl or alkoxy group containing up to four carbon atoms optionally substituted by one or more halogen atoms;
a straight- or branched chain alkenyl group containing up to six carbon atoms; or
a group -CO₂R⁵²;
R⁴ represents:
a chlorine, bromine or fluorine atom;
a straight- or branched- chain alkyl group containing up to four carbon atoms optionally substituted by one or more halogen atoms;
an alkoxy group containing up to four carbon atoms substituted by one or more halogen atoms;
-S(O)ₚR⁵³ or cyano;
R⁵ represents a straight- or branched- chain alkyl group containing up to six carbon atoms optionally substituted by one or more halogen atoms;
R⁵¹ represents ethyl or methyl;
R⁵² represents methyl or ethyl;
R⁵³ represents a straight- or branched- chain alkyl group containing up to four carbon atoms;
n represents zero, one or two; and
p represents zero, one or two.

2. A compound according to claim 1 wherein:
R³ represents a fluorine, chlorine or bromine atom; a methyl or ethyl group; an alkoxy group containing one or two carbon atoms optionally substituted by one or more halogen atoms; an alkenyl group containing from two to four carbon atoms; or -CO₂R⁵²; and
R⁴ represents a fluorine, chlorine or bromine atom; an alkyl group containing one or two carbon atoms substituted by one or more halogen atoms; an alkoxy group containing one or two carbon atoms substituted by one or more halogen atoms; or -S(O)ₚR⁵³, wherein p represents zero and R⁵³ is a methyl or ethyl group.

3. A compound according to claim 1 or 2 wherein:
R³ represents a fluorine, chlorine or bromine atom; a methyl, methoxy or ethoxy group; an alkenyl group containing two or three carbon atoms; or -CO₂R⁵² wherein R⁵² is methyl;
R⁴ represents a fluorine, chlorine or bromine atom or a group selected from trifluoromethyl, trifluoromethoxy and -S(O)ₚMe wherein p is zero; and
R⁵ represents a methyl or ethyl group.

4. A compound according to any one of the preceding claims wherein:
R³ represents fluorine, chlorine, bromine, methyl or methoxy;
R⁴ represents fluorine, chlorine, bromine or trifluoromethyl;
R⁵ represents methyl or ethyl; and
R⁵¹ represents methyl.

5. A compound according to any one of the preceding claims wherein:
R³ represents fluorine, chlorine, bromine or methoxy;
R⁴ represents fluorine, chlorine, bromine or trifluoromethyl;
R⁵ represents methyl or ethyl; and
R⁵¹ represents methyl.

6. A compound according to any one of the preceding claims wherein:
R³ represents a chlorine, bromine or fluorine atom;
R⁴ represents fluorine, chlorine, bromine or trifluoromethyl;
R⁵ represents methyl or ethyl; and
R⁵¹ represents methyl.

7. A compound according to claim 1 which is
5-cyclopropyl-4-[3,4-difluoro-2-(methylsulphonyl)benzoyl]isoxazole;
5-cyclopropyl-4-[3,4-dichloro-2-(methylsulphenyl) benzoyl]isoxazole;
5-cyclopropyl-4-[3,4-dichloro-2-(methylsulphinyl)benzoyl]isoxazole;
5-cyclopropyl-4-[3,4-dichloro-2-(methylsulphonyl)benzoyl]isoxazole;
5-cyclopropyl-4-[4-bromo-3-methoxy-2-(methylsulphenyl)benzoyl]isoxazole;
5-cyclopropyl-4-[4-bromo-3-methoxy-2-(methylsulphonyl)benzoyl]isoxazole;
5-cyclopropyl-4-[4-bromo-3-methoxy-2-(methylsulphinyl)benzoyl]isoxazole;
ethyl 5-cyclopropyl-4-[3,4-dichloro-2-(methylsulphinyl) benzoyl]isoxazole-3-carboxylate;
4-[4-chloro-3-methoxy-2-(methylsulphenyl)benzoyl]-5-cyclopropylisoxazole; or
ethyl 5-cyclopropyl-4-[3,4-dichloro-2-(methylsulphonyl)benzoyl]isoxazole-3-carboxylate.

8. The compound according to claim 1 which is ethyl 5-cyclopropyl-4-[3,4-dichloro-2-(methylsulphenyl)benzoyl]isoxazole-3-carboxylate.

9. A compound according to claim 1 which is:
4-[4-chloro-3-methoxy-2-(methylsulphinyl)benzoyl]-5-cyclopropylisoxazole;
4-[4-chloro-3-methoxy-2-(methylsulphonyl)benzoyl]-5-cyclopropylisoxazole;
4-[4-chloro-3-methyl-2-(methylsulphenyl)benzoyl]-5-cyclopropylisoxazole;
4-[4-chloro-3-fluoro-2-(methylsulphenyl)benzoyl]-5-cyclopropylisoxazole;
4-[4-chloro-3-fluoro-2-(methylsulphinyl)benzoyl]-5-cyclopropylisoxazole;
4-[4-chloro-3-fluoro-2-(methylsulphonyl)benzoyl]-5-cydopropylisoxazole;
4-[4-chloro-3-methyl-2-(methylsulphinyl)benzoyl]-5-cydopropylisoxazole;
4-[4-chloro-3-methyl-2-(methylsulphonyl)benzoyl]-5-cyclopropylisoxazole;
5-cyclopropyl-4-[3-methoxycarbonyl-2-(methylsulphenyl)-4-trifluoromethyl-benzoyl]isoxazole;
4-[4-chloro-3-methoxycarbonyl-2-(methylsulphenyl)benzoyl]-5-cyclopropyl-isoxazole;
4-[4-bromo-3-chloro-2-(methylsulphenyl)benzoyl]-5-cyclopropylisoxazole;
4-[4-bromo-3-chloro-2-(methylsulphinyl)benzoyl]-5-cyclopropylisoxazole;
4-[4-bromo-3-chloro-2-(methylsulphonyl)benzoyl]-5-cyclopropylisoxazole;
4-[4-chloro-3-methoxycarbonyl-2-(methylsulphinyl)benzoyl]-5-cyclopropyl-isoxazole;
4-[4-chloro-3-methoxycarbonyl-2-(methylsulphonyl)benzoyl]-5-cyclopropyl-isoxazole;
4-[3-chloro-2-(methylsulphenyl)-4-trifluoromethylbenzoyl]-5-cyclopropyl-isoxazole;
4-[3-chloro-2-(methylsulphonyl)-4-trifluoromethylbenzoyl]-5-cyclopropyl-isoxazole;
4-[4-bromo-3-fluoro-2-(methylsulphenyl)benzoyl]-5-cyclopropylisoxazole;
4-[4-bromo-3-fluoro-2-(methylsulphinyl)benzoyl]-5-cyclopropylisoxazole;
4-[4-bromo-3-fluoro-2-(methylsulphonyl)benzoyl]-5-cyclopropylisoxazole;
4-[4-chloro-3-isopropenyl-2-(methylsulphenyl)-benzoyl]-5-cyclopropyl-isoxazole;
5-cyclopropyl-4-[3-methyl-2,4-bis(methylsulphenyl)-benzoyl]isoxazole;
4-[4-chloro-3-isopropenyl-2-(methylsulphinyl)-benzoyl]-5-cyclopropylisoxazole; or
4-[4-chloro-3-isopropenyl-2-(methylsulphonyl)benzoyl]-5-cyclopropylisoxazole.

10. A process for the preparation of a compound of general formula (I) as defined in claim 1 which comprises:
a) the reaction of a compound of general formula (II): 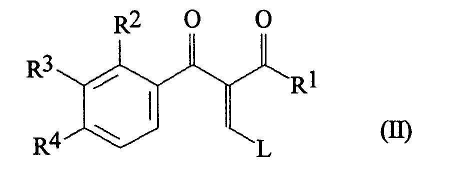 wherein R¹, R², R³ and R⁴ are as defined in claim 1 and L is a leaving group, with a salt of hydroxylamine;
b) where R represents hydrogen, R² represents a group - SR⁵¹ and R⁴ represents a group R⁴¹ which is as defined for R⁴ provided that p is zero, the reaction of a compound of general formula (III): 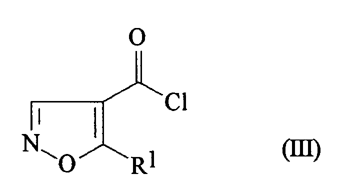 wherein R¹ is as defined in claim 1, with a compound of general formula (IV): 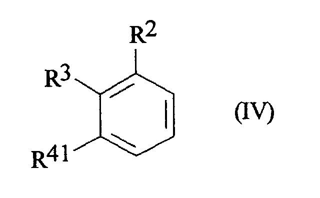 wherein R² represents a group -SR⁵¹, R³ is as defined in claim 1 and R⁴¹ is as defined above;
c) when R is hydrogen, the reaction of a compound of general formula (V): 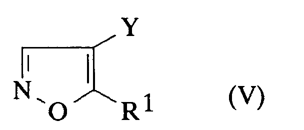 wherein R¹ is as defined in claim 1 and Y represents a carboxy group or a reactive derivative thereof, or a cyano group, with an appropriate organometallic reagent;
d) where n is one or two and/or p is one or two the oxidation of the sulphur atom of the corresponding compound of general formula (I) in which n is zero or one and/or p is zero or one.
e) where R represents a group -CO₂R⁵, n is zero or two and R⁴ represents a group R⁴² which is as defined for R⁴ provided that p is 0 or 2, the reaction of a compound of general formula (VI): 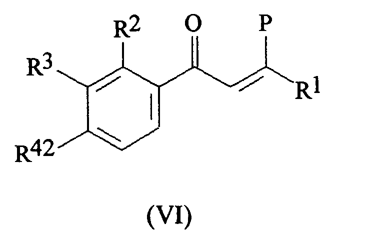 wherein R¹, R², R³ are as defined in claim 1, n is zero or two, R⁴² is as defined above and P is a leaving group, with a compound of general formula R⁵O₂CC(X) = NOH wherein R⁵ is as defined in claim 1 and X is a halogen atom;
f) where R represents a group -CO₂R⁵, n is zero or two and R⁴ represents a group R⁴², the reaction of a compound of general formula (VII): 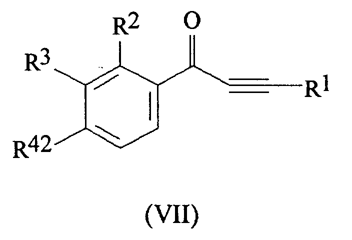 wherein R¹, R² and R³ are as defined in claim 1, n is 0 or 2 and R⁴² is as defined above, with a compound of general formula R⁵O₂CC(X) = NOH, wherein R⁵ is as defined in claim 1 and X is a halogen atom; or
g) where R represents -CO₂R⁵, n is zero or two and R⁴ represents a group R⁴², the reaction of a salt of a compound of general formula (VIII): 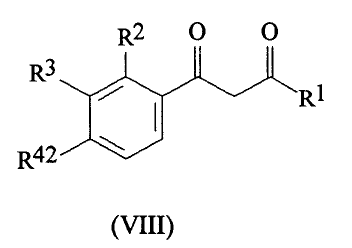 wherein R¹, R² and R³ are as defined in claim 1, n is 0 or 2 and R⁴² is as defined above, with a compound of general formula R⁵O₂CC(X) = NOH wherein R⁵ is as defined in claim 1 and X is a halogen atom.

11. A herbicidal composition which comprises as active ingredient a herbicidally effective amount of an isoxazole derivative of general formula (I) as defined in any one of claims 1 to 9 in association with an agriculturally acceptable diluent or carrier and/or surface active agent.

12. A herbicidal composition according to claim 11 which comprises 0.05 to 90% by weight of active ingredient.

13. A herbicidal composition according to claim 11 which is in liquid form and contains from 0.05 to 25% of surface-active agent.

14. A herbicidal composition according to claim 11 in the form of an aqueous suspension concentrate, a wettable powder, a water soluble or water dispersible powder, a liquid water soluble concentrate, a liquid emulsifiable suspension concentrate, a granule or an emulsifiable concentrate.

15. A method for controlling the growth of weeds at a locus which comprises applying to the locus a herbicidally effective amount of an isoxazole derivative of general formula (I) as defined in any one of claims 1 to 9.

16. A method according to claim 15 in which the locus is an area used, or to be used, for growing of crops and the compound is applied at an application rate from 0.01 kg to 4.0 kg per hectare.

17. A method according to claim 15 in which the locus is an area which is not a crop-growing area and the compound is applied at an application rate from 1.0 kg to 20.0 kg per hectare.

## Patentansprüche

1. 4-Benzoylisoxazol-Derivat der allgemeinen Formel (I): 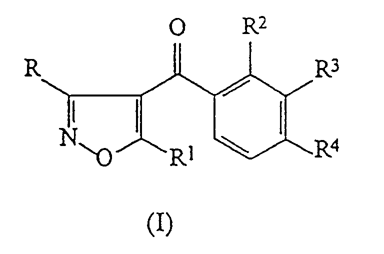 in der bedeuten:
R ein Wasserstoffatom oder eine Gruppe -CO₂R⁵,
R¹ Cyclopropyl,
R² -S(O)ₙR⁵¹,
R³ ein Chlor-, Brom- oder Fluoratom,
eine geradkettige oder verzweigte Alkyl- oder Alkoxygruppe, die bis zu 4 Kohlenstoffatome enthält, welche gegebenenfalls mit einem oder mehreren Halogenatomen substituiert ist,
eine geradkettige oder verzweigte Alkenylgruppe, die bis zu 6 Kohlenstoffatome enthält, oder
eine Gruppe -CO₂R⁵²,
R⁴ ein Chlor-, Brom- oder Fluoratom,
eine geradkettige oder verzweigte Alkylgruppe, die bis zu 4 Kohlenstoffatome enthält, welche gegebenenfalls mit einem oder mehreren Halogenatomen substituiert ist,
eine Alkoxygruppe, die bis zu 4 Kohlenstoffatome enthält, welche mit einem oder mehreren Halogenatomen substituiert ist,
-S(O)ₚR⁵³ oder Cyano,
R⁵ eine geradkettige oder verzweigte Alkylgruppe, die bis zu 6 Kohlenstoffatome enthält, welche gegebenenfalls mit einem oder mehreren Halogenatomen substituiert ist,
R⁵¹ Ethyl oder Methyl,
R⁵² Methyl oder Ethyl,
R⁵³ eine geradkettige oder verzweigte Alkylgruppe, die bis zu 4 Kohlenstoffatome enthält,
n 0, 1 oder 2 und
p 0, 1 oder 2.

2. Verbindung nach Anspruch 1, wobei
R³ ein Fluor-, Chlor- oder Bromatom, eine Methyl- oder Ethylgruppe, eine Alkoxygruppe, die ein oder zwei Kohlenstoffatome enthält, welche gegebenenfalls mit einem oder mehreren Halogenatomen substituiert ist, eine Alkenylgruppe, die 2 bis 4 Kohlenstoffatome enthält, oder -CO₂R⁵²- bedeutet, und
R⁴ ein Fluor-, Chlor- oder Bromatom, eine Alkylgruppe, die 1 oder 2 Kohlenstoffatome enthält, welche mit einem oder mehreren Halogenatomen substituiert ist, eine Alkoxygruppe, die 1 oder 2 Kohlenstoffatome enthält, welche mit einem oder mehreren Halogenatomen substituiert ist, oder -S(O)ₚR⁵³ bedeutet, wobei p 0 ist und R⁵³ eine Methyl- oder Ethylgruppe ist.

3. Verbindung nach Anspruch 1 oder 2, wobei
R³ ein Fluor-, Chlor- oder Bromatom, eine Methyl-, Methoxy- oder Ethoxygruppe, eine Alkenylgruppe, die 2 oder 3 Kohlenstoffatome enthält, oder -CO₂R⁵² darstellt, wobei R⁵² Methyl ist,
R⁴ ein Fluor-, Chlor- oder Bromatom oder eine unter Trifluormethyl, Trifluormethoxy und -S(O)ₚMe ausgewählte Gruppe darstellt, wobei p 0 ist und R⁵ eine Methyl- oder Ethylgruppe bedeutet.

4. Verbindung nach einem der vorhergehenden Ansprüche, wobei
R³ Fluor, Chlor, Brom, Methyl oder Methoxy bedeutet,
R⁴ Fluor, Chlor, Brom oder Trifluormethyl bedeutet,
R⁵ Methyl oder Ethyl bedeutet und
R⁵¹ Methyl bedeutet.

5. Verbindung nach einem der vorhergehenden Ansprüche, wobei
R³ Fluor, Chlor, Brom oder Methoxy bedeutet,
R⁴ Fluor, Chlor, Brom oder Trifluormethyl bedeutet,
R⁵ Methyl oder Ethyl bedeutet und
R⁵¹ Methyl bedeutet.

6. Verbindung nach einem der vorhergehenden Ansprüche, wobei
R³ ein Chlor-, Brom- oder Fluoratom bedeutet,
R⁴ Fluor, Chlor, Brom oder Trifluormethyl bedeutet,
R⁵ Methyl oder Ethyl bedeutet und
R⁵¹ Methyl bedeutet.

7. Verbindung nach Anspruch 1, nämlich:
5-Cyclopropyl-4-[3,4-difluor-2-(methylsulfonyl)benzoyl]isoxazol;
5-Cyclopropyl-4-[3,4-dichlor-2-(methylsulfenyl)benzoyl]isoxazol;
5-Cyclopropyl-4-[3,4-dichlor-2-(methylsulfinyl)benzoyl]isoxazol;
5-Cyclopropyl-4-[3,4-dichlor-2-(methylsulfonyl)benzoyl]isoxazol;
5-Cyclopropyl-4-[4-brom-3-methoxy-2-(methylsulfenyl)benzoyl]isoxazol;
5-Cyclopropyl-4-[4-brom-3-methoxy-2-(methylsulfonyl)benzoyl]isoxazol;
5-Cyclopropyl-4-[4-brom-3-methoxy-2-(methylsulfinyl)benzoyl]isoxazol;
5-Cyclopropyl-4-[3,4-dichlor-2-(methylsulfinyl)benzoyl]isoxazol-3-carbonsäureethylester;
4-[4-Chlor-3-methoxy-2-(methylsulfenyl)benzoyl]-5-cyclopropylisoxazol oder
5-Cyclopropyl-4-[3,4-dichlor-2-(methylsulfonyl)benzoyl]isoxazol-3-carbonsäureethylester.

8. Verbindung nach Anspruch 1, nämlich:
5-Cyclopropyl-4-[3,4-dichlor-2-(methylsulfenyl)benzoyl]isoxazol-3-carbonsäureethylester.

9. Verbindung nach Anspruch 1, nämlich:
4-[4-Chlor-3-methoxy-2-(methylsulfinyl)benzoyl]-5-cyclopropylisoxazol;
4-[4-Chlor-3-methoxy-2-(methylsulfonyl)benzoyl]-5-cyclopropylisoxazol;
4-[4-Chlor-3-methyl-2-(methylsulfenyl)benzoyl]-5-cyclopropylisoxazol;
4-[4-Chlor-3-fluor-2-(methylsulfenyl)benzoyl]-5-cyclopropylisoxazol;
4-[4-Chlor-3-fluor-2-(methylsulfinyl)benzoyl]-5-cyclopropylisoxazol;
4-[4-Chlor-3-fluor-2-(methylsulfonyl)benzoyl]-5-cyclopropylisoxazol;
4-[4-Chlor-3-methyl-2-(methylsulfinyl)benzoyl]-5-cyclopropylisoxazol;
4-[4-Chlor-3-methyl-2-(methylsulfonyl)benzoyl]-5-cyclopropylisoxazol;
5-Cyclopropyl-4-[3-methoxycarbonyl-2-(methylsulfenyl)-4-trifluormethyl-benzoyl]isoxazol;
4-[4-Chlor-3-methoxycarbonyl-2-(methylsulfenyl)benzoyl]-5-cyclopropyl-isoxazol;
4-[4-Brom-3-chlor-2-(methylsulfenyl)benzoyl]-5-cyclopropylisoxazol;
4-[4-Brom-3-chlor-2-(methylsulfinyl)benzoyl]-5-cyclopropylisoxazol;
4-[4-Brom-3-chlor-2-(methylsulfonyl)benzoyl]-5-cyclopropylisoxazol;
4-[4-Chlor-3-methoxycarbonyl-2-(methylsulfinyl)benzoyl]-5-cyclopropyl-isoxazol;
4-[4-Chlor-3-methoxycarbonyl-2-(methylsulfonyl)benzoyl]-5-cyclopropyl-isoxazol;
4-[3-Chlor-2-(methylsulfenyl)-4-trifluormethylbenzoyl]-5-cyclopropyl-isoxazol;
4-[3-Chlor-2-(methylsulfonyl)-4-trifluormethylbenzoyl]-5-cyclopropyl-isoxazol;
4-[4-Brom-3-fluor-2-(methylsulfenyl)benzoyl]-5-cyclopropylisoxazol;
4-[4-Brom-3-fluor-2-(methylsulfinyl)benzoyl]-5-cyclopropylisoxazol;
4-[4-Brom-3-fluor-2-(methylsulfonyl)benzoyl]-5-cyclopropylisoxazol;
4-[4-Chlor-3-isopropenyl-2-(methylsulfenyl)benzoyl]-5-cyclopropyl-isoxazol;
5-Cyclopropyl-4-[3-methyl-2,4-bis(methylsulfenyl)benzoyl]isoxazol;
4-[4-Chlor-3-isopropenyl-2-(methylsulfinyl)benzoyl]-5-cyclopropylisoxazol oder
4-[4-Chlor-3-isopropenyl-2-(methylsulfonyl)benzoyl]-5-cyclopropylisoxazol.

10. Verfahren zur Herstellung einer Verbindung der in Anspruch 1 definierten allgemeinen Formel (I), das umfaßt:
a) die Umsetzung einer Verbindung der allgemeinen Formel (II): 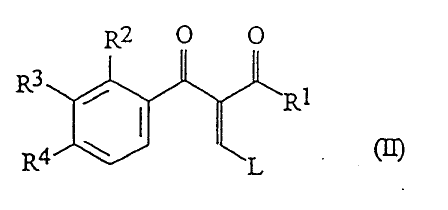 in der R¹, R², R³ und R⁴ wie in Anspruch 1 definiert sind und L eine Austrittsgruppe ist, mit einem Salz von Hydroxylamin,
b) wenn R Wasserstoff, R² eine Gruppe -SR⁵¹ und R⁴ eine Gruppe R⁴¹ darstellt, die wie für R⁴ definiert ist, mit der Maßgabe, daß p 0 ist, die Umsetzung einer Verbindung der allgemeinen Formel (III): 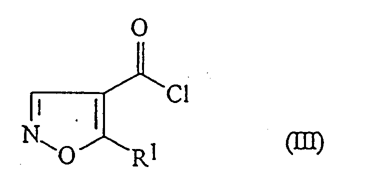 in der R¹ wie in Anspruch 1 definiert ist, mit einer Verbindung der allgemeinen Formel (IV): 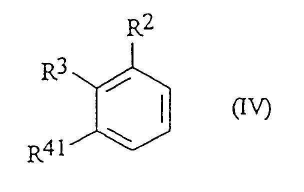 in der R² eine Gruppe -SR⁵¹ bedeutet, R³ wie in Anspruch 1 definiert ist und R⁴¹ wie oben definiert ist,
c) wenn R Wasserstoff ist, die Umsetzung einer Verbindung der allgemeinen Formel (V): 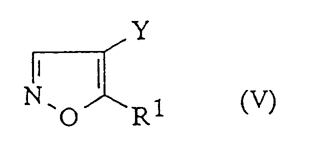 in der R¹ wie in Anspruch 1 definiert ist und Y eine Carboxygruppe oder ein reaktives Derivat davon oder eine Cyanogruppe darstellt, mit einem geeigneten organometallischen Reagens,
d) wenn n 1 oder 2 und/oder p 1 oder 2 ist, die Oxidation des Schwefelatoms der entsprechenden Verbindung der allgemeinen Formel (I), in der n 0 oder 1 und/oder p 0 oder 1 ist,
e) wenn R eine Gruppe -CO₂R⁵ darstellt, n 0 oder 2 ist und R⁴ eine Gruppe R⁴² darstellt, die wie für R⁴ definiert ist, mit der Maßgabe, daß p 0 oder 2 ist, die Umsetzung einer Verbindung der allgemeinen Formel (VI): 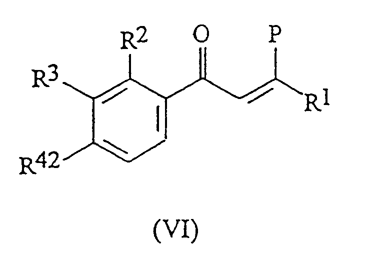 in der R¹, R² und R³ wie in Anspruch 1 definiert sind, n 0 oder 2 ist, R⁴² wie oben definiert ist und P eine Austrittsgruppe ist, mit einer Verbindung der allgemeinen Formel R⁵O₂CC(X)=NOH, wobei R⁵ wie in Anspruch 1 definiert ist und X ein Halogenatom ist,
f) wenn R eine Gruppe -CO₂R⁵ bedeutet, n 0 oder 2 ist und R⁴ eine Gruppe R⁴² bedeutet, die Umsetzung einer Verbindung der allgemeinen Formel (VII): 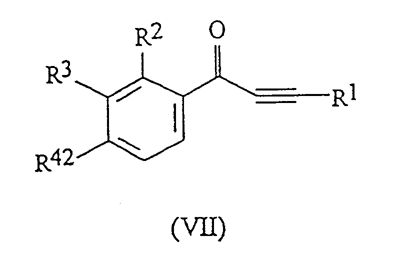 in der R¹, R² und R³ wie in Anspruch 1 definiert sind, n 0 oder 2 ist und R⁴² wie oben definiert ist, mit einer Verbindung der allgemeinen Formel R⁵O₂CC(X)=NOH, wobei R⁵ wie in Anspruch 1 definiert ist und X ein Halogenatom ist, oder
g) wenn R -CO₂R⁵ bedeutet, n 0 oder 2 ist und R⁴ eine Gruppe R⁴² bedeutet, die Umsetzung eines Salzes einer Verbindung der allgemeinen Formel (VIII): 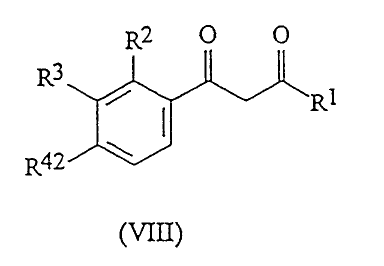 in der R¹, R² und R³ wie in Anspruch 1 definiert sind, n 0 oder 2 ist und R⁴² wie oben definiert ist, mit einer Verbindung der allgemeinen Formel R⁵O₂CC(X)=NOH, wobei R⁵ wie in Anspruch 1 definiert ist und X ein Halogenatom ist.

11. Herbicide Zusammensetzung, die als Wirkstoff eine herbicid wirksame Menge eines in einem der Ansprüche 1 bis 9 definierten Isoxazol-Derivats der allgemeinen Formel (I) in Verbindung mit einem landwirtschaftlich akzeptablen Verdünnungsmittel oder Träger und/oder grenzflächenaktiven Mittel enthält.

12. Herbicide Zusammensetzung nach Anspruch 11, die 0,05 bis 90 Gew.-% Wirkstoff enthält.

13. Herbicide Zusammensetzung nach Anspruch 11, die in flüssiger Form vorliegt und 0,05 bis 25 % grenzflächenaktives Mittel enthält.

14. Herbicide Zusammensetzung nach Anspruch 11 in Form von wäßrigen Suspensionskonzentraten, benetzbaren Pulvern, wasserlöslichen oder in Wasser dispergierbaren Pulvern, flüssigen wasserlöslichen Konzentraten, flüssigen, emulgierbaren Suspensionskonzentraten, Körnern oder emulgierbaren Konzentraten.

15. Verfahren zur Bekämpfung des Wachstums von Unkräutern an einer Örtlichkeit, bei dem auf die Örtlichkeit eine herbicid wirksame Menge eines in einem der Ansprüche 1 bis 9 definierten Isoxazol-Derivats der allgemeinen Formel (I) ausgebracht wird.

16. Verfahren nach Anspruch 15, wobei die Örtlichkeit eine Fläche ist, die zum Anbau von Kulturpflanzen verwendet wird oder verwendet werden soll, und die Verbindung in einer Ausbringmenge von 0,01 kg bis 4,0 kg/ha ausgebracht wird.

17. Verfahren nach Anspruch 15, wobei die Örtlichkeit eine Fläche ist, auf der keine Kulturpflanzen angebaut werden, und die Verbindung in einer Ausbringmenge von 1,0 kg bis 20,0 kg/ha ausgebracht wird.

## Revendications

1. Un dérivé du 4-benzoyl isoxazole répondant à la formule générale (I) : 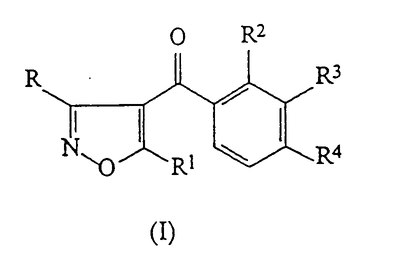 dans laquelle :
R représente l'hydrogène ou un radical -CO₂R⁵;
R¹ représente un radical cyclopropyle ;
R² représente un radical -S(O)ₙR⁵¹
R³ représente :
un atome de chlore, de brome ou de fluor ;
un radical alkyle ou alkoxy, linéaire ou ramifié, contenant de 1 à 4 atomes de carbone, éventuellement substitué par un ou plusieurs atomes d'halogènes ;
un radical alkényle linéaire ou ramifié, contenant de 1 à 6 atomes de carbone ; ou
un radical -CO₂R⁵² ;
R⁴ représente :
un atome de chlore, de brome ou de fluor ;
un radical alkyle, linéaire ou ramifié, contenant de 1 à 4 atomes de carbone, éventuellement substitué par un ou plusieurs atomes d'halogènes ;
un radical alkoxy, linéaire ou ramifié, contenant de 1 à 4 atomes de carbone, substitué par un ou plusieurs atomes d'halogènes ;
un radical -S(O)ₚR⁵³ ou cyano ;
R⁵ représente :
un radical alkyle linéaire ou ramifié contenant de 1 à 6 atomes de carbone, éventuellement substitué par un ou plusieurs atomes d'halogènes ;
R⁵¹ représente un radical méthyle ou éthyle ;
R⁵² représente un radical méthyle ou éthyle ;
R⁵³ représente un radical alkyle linéaire ou ramifié contenant de 1 à 4 atomes de carbone ;
n représente 0, 1 ou 2 ; et
p représente 0, 1 ou 2

2. Un composé selon la revendication 1, dans laquelle :
R³ représente un atome de fluor, de chlore ou de brome ; un radical méthyle ou éthyle ; un radical alkoxy contenant 1 ou 2 atomes de carbone éventuellement substitué par un ou plusieurs atomes d'halogènes un radical alkényle contenant de 2 à 4 atomes de carbone ; ou un radical -CO₂R⁵² ; et
R⁴ représente un atome de fluor, de chlore ou de brome ; un radical alkyle contenant 1 ou 2 atomes de carbone substitué par un ou plusieurs atomes d'halogène ; un radical alkoxy contenant 1 ou 2 atomes de carbone substitué par un ou plusieurs atomes d'halogènes ; ou un radical -S(O)ₚR⁵³, dans lequel p représente zéro et R⁵³ représente un radical méthyle ou un radical éthyle.

3. Un composé selon la revendication 1 ou 2, dans laquelle :
R³ représente un atome de fluor, de chlore ou de brome ; un radical méthyle, méthoxy ou éthoxy ; un radical alkényle contenant 2 ou 3 atomes de carbone ; ou un radical -CO₂R⁵² dans lequel R⁵² représente un radical méthyle ;
R⁴ représente un atome de fluor, de chlore ou de brome ou un radical choisi parmi les radicaux trifluorométhyle, trifluorométhoxy et -S(O)ₚMe dans lequel p représente zéro ; et
R⁵ représente un radical méthyle ou éthyle.

4. Un composé selon l'une quelconque des revendications précédentes dans laquelle :
R³ représente un atome de fluor, de chlore ou de brome ou un radical méthyle ou méthoxy ;
R⁴ représente un atome de fluor, de chlore ou de brome ou un radical trifluorométhyle ;
R⁵ représente un radical méthyle ou éthyle ; et
R⁵¹ représente un radical méthyle.

5. Un composé selon l'une quelconque des revendications précédentes dans laquelle :
R³ représente un atome de fluor, de chlore ou de brome ou un radical méthoxy ;
R⁴ représente un atome de fluor, de chlore ou de brome ou un radical trifluorométhyle ;
R⁵ représente un radical méthyle ou éthyle ; et
R⁵¹ représente un radical méthyle.

6. Un composé selon l'une quelconque des revendications précédentes dans laquelle :
R³ représente un atome de fluor, de chlore ou de brome ;
R⁴ représente un atome de fluor, de chlore ou de brome ou un radical trifluorométhyle ;
R⁵ représente un radical méthyle ou éthyle ; et
R⁵¹ représente un radical méthyle.

7. Un composé selon la revendication 1. qui est le :
5-cyclopropyl-4-[3,4-difluoro-2-(méthylsulfonyl)benzoyl]- isoxazole ;
5-cyclopropyl-4-[3,4-dichloro-2-(méthylsulfényl)benzoyl]- isoxazole ;
5-cyclopropyl-4-[3,4-dichloro-2-(méthylsulfinyl)benzoyl]- isoxazole ;
5-cyclopropyl-4-[3,4-dichloro-2-(méthylsulfonyl)benzoyl]- isoxazole ;
5-cyclopropyl-4-[4-bromo-3-méthoxy-2-(méthylsulfényl)benzoyl]-isoxazole ;
5-cyclopropyl-4-[4-bromo-3-méthoxy-2-(méthylsulfonyl)benzoyl]-isoxazole ;
5-cyclopropyl-4-[4-bromo-3-méthoxy-2-(méthylsulfinyl)benzoyl]-isoxazole ;
5-cyclopropyl-4-[3,4-dichloro-2-(méthylsulfinyl)benzoyl]- isoxazole-3-carboxylate d'éthyle ;
4-[4-chloro-3-méthoxy-2-(méthylsulfényl)benzoyl]-5-cyclopropyl isoxazole ;
5-cyclopropyl-4-[3,4-dichloro-2-(méthylsulfonyl)benzoyl]- isoxazole-3-carboxylate d'éthyle ;

8. Un composé selon la revendication 1. qui est le
5-cyclopropyl-4-[3,4-dichloro-2-(méthylsulfényl)benzoyl]- isoxazole-3-carboxylate d'éthyle ;

9. Un composé selon la revendication 1. qui est:
4-[4-chloro-3-méthoxy-2-(méthylsulfinyl)benzoyl]-5-cyclopropyl isoxazole ;
4-[4-chloro-3-méthoxy-2-(méthylsulfonyl)benzoyl]-5-cyclopropyl isoxazole ;
4-[4-chloro-3-méthyl-2-(méthylsulfényl)benzoyl]-5-cyclopropyl isoxazole ;
4-[4-chloro-3-fluoro-2-(méthylsulfényl)benzoyl]-5-cyclopropyl isoxazole ;
4-[4-chloro-3-fluoro-2-(méthylsulfinyl)benzoyl]-5-cyclopropyl isoxazole ;
4-[4-chloro-3-fluoro-2-(méthylsulfonyl)benzoyl]-5-cyclopropyl isoxazole ;
4-[4-chloro-3-méthyl-2-(méthylsulfinyl)benzoyl]-5-cyclopropyl isoxazole ;
4-[4-chloro-3-méthyl-2-(méthylsulfonyl)benzoyl]-5-cyclopropyl isoxazole ;
5-cyclopropyl-4-[3-méthoxycarbonyl-2-(méthylsulfényl)-4-trifluorométhylebenzoyl]-isoxazole ;
4-[4-chloro-3-méthoxycarbonyl-2-(méthylsulfényl)benzoyl-]- 5-cyclopropyl isoxazole ;
4-[4-bromo-3-chloro-2-(méthylsulfényl)benzoyl-]- 5-cyclopropyl isoxazole ;
4-[4-bromo-3-chloro-2-(méthylsulfinyl)benzoyl-]- 5-cyclopropyl isoxazole ;
4-[4-bromo-3-chloro-2-(méthylsulfonyl)benzoyl-]- 5-cyclopropyl isoxazole ;
4-[4-chloro-3-méthoxycarbonyl-2-(méthylsulfinyl)benzoyl-]- 5-cyclopropyl isoxazole ;
4-[4-chloro-3-méthoxycarbonyl-2-(méthylsulfonyl)benzoyl-]- 5-cyclopropyl isoxazole ;
4-[3-chloro-2-(méthylsulfényl)-4-trifluorométhylbenzoyl-]- 5-cyclopropyl isoxazole ;
4-[3-chloro-2-(méthylsulfonyl)-4-trifluorométhylbenzoyl-]- 5-cyclopropyl isoxazole ;
4-[4-bromo-3-fluoro-2-(méthylsulfényl)benzoyl-]- 5-cyclopropyl isoxazole ;
4-[4-bromo-3-fluoro-2-(méthylsulfinyl)benzoyl-]- 5-cyclopropyl isoxazole ;
4-[4-bromo-3-fluoro-2-(méthylsulfonyl)benzoyl-]- 5-cyclopropyl isoxazole ;
4-[4-chloro-3-isopropényl-2-(méthylsulfényl)benzoyl-]- 5-cyclopropyl isoxazole ;
5-cyclopropyl-4-[3-méthyl-2,4-bis(méthylsulfényl)-benzoyl]-isoxazole
4-[4-chloro-3-isopropényl-2-(méthylsulfinyl)benzoyl-]- 5-cyclopropyl isoxazole ; ou
4-[4-chloro-3-isopropényl-2-(méthylsulfonyl)benzoyl-]- 5-cyclopropyl isoxazole.

10. Un procédé de préparation d'un composé de formule générale (I) tel que défini dans la revendication 1, qui comprend:
a) action d'un composé de formule générale (II) : 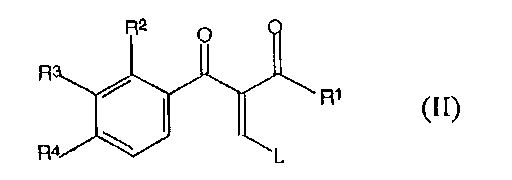 dans laquelle R¹, R², R³ et R⁴ ont la définition donnée dans la revendication 1 et L représente un groupe partant, sur un sel d'hydroxylamine ;
b) Si R représente l'hydrogène, R² représente un groupe -SR⁵¹, et R⁴ représente un radical R⁴¹ qui a la définition donnée pour R⁴ à condition que p soit égal à zéro, action d'un composé de formule générale (III) : 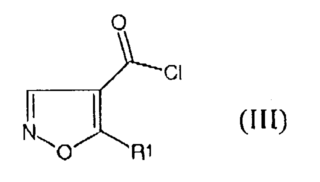 dans laquelle R¹ a la définition donnée dans la revendication 1, sur un composé de formule générale (IV) : 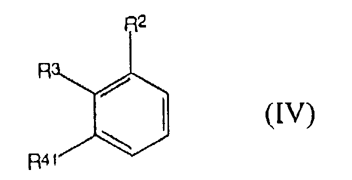 dans laquelle R² représente -SR⁵¹, R³ a la définition donnée dans la revendication 1 et R⁴¹ a la définition donnée ci-dessus ;
c) Si R représente l'hydrogène, action d'un composé de formule générale (V) : 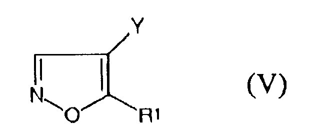 dans laquelle R¹ a la définition donnée dans la revendication 1 et Y représente un radical carboxy ou un dérivé réactif d'un radical carboxy, ou un radical cyano, sur un réactif organométallique approprié ;
d) si n représente 1 ou 2 et / ou p représente 1 ou 2, oxydation de l'atome de soufre du composé correspondant de formule générale (I) dans lequel n représente 0 ou 1 et / ou p représente 0 ou 1 ;
e) si R représente un radical -CO₂R⁵, n représente 0 ou 2 et R⁴ représente un radical R⁴² qui a la définition donnée pour R⁴ à condition que p représente 0 ou 2, action d'un composé de formule générale (VI) : 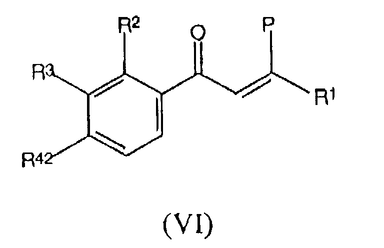 dans laquelle R¹, R², R³ ont la définition donnée dans la revendication 1, n représente 0 ou 2, R⁴² a la définition donnée ci-dessus, et P représente un groupe partant, sur un composé de formule générale R₅O₂CC(X)=NOH dans laquelle R⁵ a la définition donnée dans la revendication 1 et X représente un atome d'halogène ;
f) si R représente un groupe -CO₂R⁵, n représente 0 ou 2 et R⁴ représente un radical R⁴², action d'un composé de formule générale (VII) : 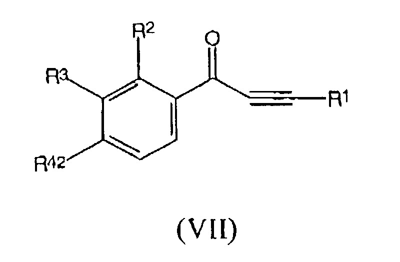 dans laquelle R¹, R², R³ sont tels que définis dans la revendication 1, n a la valeur 0 ou 2 et R⁴² a la définition donnée ci-dessus, sur un composé de formule générale R⁵O₂CC(X)=NOH dans laquelle R⁵ a la définition donnée dans la revendication 1 et X représente un atome d'halogène ; ou
g) si R représente -CO₂R⁵, n représente 0 ou 2 et R⁴ représente un radical R⁴², action d'un sel d'un composé de formule générale (VIII) : 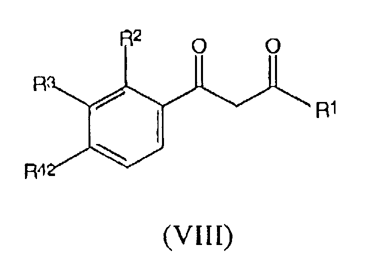 dans laquelle R¹, R² et R³ ont la définition donnée dans la revendication 1, n représente 0 ou 2 et R⁴² a la définition donnée ci-dessus, sur un composé de formule générale R⁵O₂CC(X)=NOH dans laquelle R⁵ a la définition donnée dans la revendication 1 et X représente un atome d'halogène.

11. Une composition herbicide, qui contient comme matière active une quantité herbicide efficace d'un dérivé de l'Isoxazole de formule générale (I) comme défini dans l'une quelconque des revendications 1 à 9, associée avec un diluant ou une charge et / ou un agent tensioactif acceptable pour des usages agricoles.

12. Une composition herbicide selon la revendication 11, qui contient de 0,05 à 90 % en poids de matière active.

13. Une composition herbicide selon la revendication 11, qui se présente sous forme liquide et contient 0,05 à 25 % en poids d'agent tensioactif.

14. Une composition herbicide selon la revendication 11, qui se présente sous la forme d'une suspension aqueuse concentrée, d'une poudre mouillable, d'une poudre soluble dans l'eau ou dispersable dans l'eau, d'un concentré liquide soluble dans l'eau, d'une suspension concentrée liquide émulsionnable, de granulés ou d'un concentré émulsionnable.

15. Un procédé de contrôle de la croissance de mauvaises herbes en un lieu qui consiste à appliquer sur le-dit lieu une quantité herbicide efficace d'un dérivé de l'isoxazole de formule générale (I) tel que défini dans l'une quelconque des revendications 1 à 9.

16. Un procédé selon la revendication 15 dans lequel le lieu est une zone utilisée, ou destinée a être utilisée pour faire pousser des cultures et dans laquelle le composé est appliqué à une dose comprise entre de 0,01 et 4,0 kg par hectare.

17. Un procédé selon la revendication 15 dans lequel le lieu est une zone non cultivée dans laquelle le composé est utilisé à une dose comprise entre 1,0 et 20,0 kg par hectare.
